# EUROPEAN PATENT APPLICATION

(11) **EP 2 594 547 A1**
(43) Date of publication of application: **22.05.2013**
(21) Application number: 11189628.8
(22) Date of filing: 17.11.2011
(51) Int. Cl.: C07C 17/02, C07C 17/156, C07C 17/25, C07C 19/045, C07C 21/06, C07C 1/24, C08F 14/06

(54) **Process for the manufacture of at least one ethylene derivative compound from bioethanol**

(71) Applicant: SOLVAY SA, 1120 Bruxelles (BE)
(72) Inventor: Strebelle, Michel, 1150 Brussels (BE); Degraeve, Paul Julius, 7822 Isières (BE)
(74) Representative: Vande Gucht, Anne

(57) **Abstract**

Process for the manufacture of at least one ethylene derivative compound starting from bioethanol comprising dehydration of bioethanol, separation and compression to form a stream A1, division of stream A1 into two slipstreams B1 and B2, wherein slipstream B 1 is fed to a first reactor to produce at least one ethylene derivative, and slipstream B2 is fed to a second reactor to produce at least one ethylene derivative after being subjected to an oxygenates removal step and a drying step. The process may optionally include a secondary compression of slipstream B2, desulfurization of stream A1, and/or separate desulfurization of slipstreams B 1 and/or B2. In preferred embodiments, the first and second reactors comprises oxychlorination and chlorination, respectively; and the ethylene derivative compound formed in both reactor is 1,2-dichloethane.

## Description

### Cross reference to related applications

Not applicable.

### Statement regarding federally sponsored research or development

Not applicable.

### Technical field of the invention

The present invention relates to a process for the manufacture of at least one ethylene derivative compound, in particular to a process for the manufacture of 1,2-dichloroethane (DCE) and optionally also of at least another ethylene derivative compound such as vinyl chloride and polyvinyl chloride, wherein the manufacturing process starts with an ethylene feedstock produced from a biomass-derived ethanol feedstock.

### Background of the invention

One of the problems faced by the manufacture of polymers from ethylene is that the starting raw materials are from fossil fuels (natural gas, crude oil) which are non-renewable feedstocks. Indeed, ethylene is an olefin mainly produced as a by-product of oil refining, through vapor reforming or catalytic cracking processes. Another route used for the ethylene production is the recovery and dehydrogenation of the ethane present in natural gas. However the reserves of fossil fuels being quite limited, the production of such fuels entails the extraction from much deeper resources which is increasingly technically challenging, costlier in energy consumption and requiring the use of very sophisticated equipment. Such constraint in needing to extract less accessible fossil matter at a higher expense causes a direct increase in the cost of manufacture of ethylene and the ethylene derivatives compounds.

Applicants however have realized that the production of ethylene and ethylene derivatives compounds would benefit by the replacement of at least a part of the carbonaceous raw materials of fossil origin by renewable resources, such as carbonaceous matter derived from biomass. Of particular interest is the ethanol feedstock which is produced from renewable resources. Such biomass-derived ethanol, also referred to as 'bioethanol' or 'hydrous fuel alcohol' can be prepared in large quantities from biomass via fermentation. The different feedstocks for producing ethanol may be sucrose-containing feedstocks (e.g., sugarcane), starchy materials (e.g., corn, starch, wheat, cassava,...), lignocellulosic biomass (e.g., switchgrass) and/or agricultural waste. The purification or isolation of bioethanol is frequently carried out by complicated, multistage distillation.

Even after the known purification processes, the advantage of bioethanol is frequently decreased by small amounts of impurities it contains. Bioethanol impurities may include oxygen-containing organics, for example other alcohols such as isopropanol, n-propanol, and isobutanol, and/or aldehydes such as acetaldehyde. Bioethanol impurities may further include sulfur-containing impurities, such as inorganic sulfur compounds (sulfite, sulfate) C₂-10-dialkyl sulfides, C₂-10-dialkyl disulfides, C₂-10-dialkyl sulfoxides, C₂-10-alkyl mercaptans, 3-methylthio-1-propanol, and/or S-containing amino acids.

Some of these impurities may interfere with the downstream processes which use bioethanol as feedstock and which generate chemical products, especially when some of the downstream steps are catalytic conversions.

Sulfur-containing impurities are known to have a negative impact on catalysts, especially those containing catalytic metals. In general, the sulfur content of chemicals derived from natural raw materials generally have an adverse effect on a reaction carried out using them, for instance as a result of metal centers being attacked by sulfur and thereby deactivated, or as a result of acidic or basic centers being occupied, may cause secondary reactions occurring or being catalyzed, formation of deposits in production plants and/or contamination of the products. For example, the sulfur content of bioethanol interferes in its use in ammination to form ethylamines by poisoning the metal catalyst. The sulfur content of bioethanol also has an adverse effect due to poisoning of catalyst, e.g., in steam reforming processes for the production of hydrogen and in fuel cells. These bioethanol impurities may be carried over and/or transformed into other sulfur-containing compounds into the processes which generate ethylene derivative compound(s) or any compound derived therefrom, examples of which include 1,2-dichloroethane, vinyl chloride, and polyvinyl chloride (PVC).

If efforts are not made to remove at least some of these impurities, the yield of desired intermediate and final products (ethylene, at least one ethylene derivative compound or any compound derived therefrom) and efficacy of the overall process may be diminished.

To date, ethylene which is typically used for the manufacture of polymers comprising ethylene and of 1, 2-dichloroethane (DCE) is more than 99.8 % pure (i.e., contains impurities of 0.2 % or less). Ethylene of very high purity is in high demand for production various polyethylene polymers, and may be sometimes termed 'polyethylene-grade' ethylene. The very high purity ethylene is generally obtained via the cracking of various petroleum products, followed by numerous complex and expensive separation operations in order to isolate the ethylene from the other products of cracking and to obtain a product of very high purity. Sulfur compounds and oxygen containing compounds can accelerate polymerization reaction of unsaturated hydrocarbons whereby resinous materials will be accumulated in reactors or pipelines risking clogging, and may act as poison for polymerization; thus these impurities are generally removed from cracked ethylene. The formation of same carbon number alkanes is known to add significantly to the complexity and cost of producing purified olefins for polymer manufacture. For example, the industrially practiced catalytic cracking of hydrocarbon feedstocks to produce olefins for polymer manufacture is a capitally intensive process with a significant proportion of the cost involved in same carbon number olefin and alkane separation.

Given the high costs linked to the production of ethylene of such high purity and to the increasing cost of the carbonaceous raw material, various processes for the manufacture of ethylene derivative compounds, in particular DCE, using ethylene having a purity of less than 99.8 % have been developed. These processes have the advantage of reducing the costs by simplifying the course of separating the products resulting from the cracking and by thus abandoning complex separations which are of no benefit for the manufacture of ethylene derivative compounds, in particular DCE.

For example, patent application WO 00/26164 describes a process for the manufacture of DCE by simplified cracking of ethane coupled with chlorination of ethylene. To this effect, an ethylene chlorination step takes place in the presence of the impurities obtained during the cracking of the ethane.

Patent application WO 03/048088 describes the production of low-concentration ethylene for the chemical reaction with chlorine by means of ethane dehydrogenation. The ethane-loaded gas stream contains not only hydrogen and methane, but also high amounts of unconverted ethane. For the economic design of the process, the unconverted ethane must be fed back to ethane dehydrogenation after complicated cleaning processes. This process can only use ethane as feedstock. A significant disadvantage is the very low concentration of ethylene in the cracked product - less than 60 % - as well as the fact that further components of the gas stream such as hydrogen, propylene, butadiene only allow to use such ethylene in very special processes.

Further, patent applications WO 2006/067188, WO 2006/067190, WO 2006/067191, WO 2006/067192, WO 2006/067193 and WO 2007/147870 describe processes for the manufacture of DCE starting from a hydrocarbon source, in particular naphtha, gas oil, natural gas liquid, ethane, propane, butane, isobutane, or mixtures thereof, which is first subjected to a simplified cracking. Two different fractions containing ethylene are afterwards separated from the gas mixture issued from the simplified cracking before being conveyed independently to a chlorination reactor and to an oxychlorination reactor in order to produce DCE.

Those processes, the aim of which is to produce and use ethylene having a purity of less than 99.8 %, present however the disadvantages of requiring a first step of cracking which needs an important investment causing an increase in the production costs and further of involving the use of expensive hydrocarbon sources.

The route from ethanol to ethylene does not involved cracking, but involves dehydration. The dehydration reaction is characterized in that carbon-carbon double bonds are formed by elimination of water only and does not include the coupling of carbon fragments.

Ethylene from bioethanol can also be prepared with a purity ranging from 80 to 97% on a water-free basis, but such process requires water removal and extensive separation for compounds lighter than ethylene (e.g., CO, CO₂ CH₄), and compounds heavier than ethylene (e.g., propylene).

Processes for dehydration of ethanol to ethylene are well known. These processes typically require temperatures in excess of 300°C where both the olefin and alcohol are in the gas phase and achieve near complete or essentially complete conversion of alcohol to olefin. The thermodynamics favor such high alcohol to olefin conversions only at low pressure, so the process is conventionally operated at or just above atmospheric pressure (e.g., 1 to 2 atm). However, renewable ethanol still contains water, and the presence of water is thermodynamically detrimental to achieving a high conversion in gas-phase ethanol-to-ethylene dehydration.

In a typical gas-phase dehydration process, ethanol is first vaporized and fed to a dehydration reactor (e.g., containing two vessels generally operated in alternating mode to allow regeneration of catalyst) at about 1 atm. Since the dehydration reaction is endothermic, there is an input of heat provided to the reactor. A hot ethylene stream exiting the dehydration reactor is quenched with water and is generally scrubbed with caustic. The ethylene produced must generally meet critical purity specifications. Purification is typically done via cryogenic distillation at elevated pressure, so when ethylene is produced by the gas phase dehydration of ethanol, it must be compressed before purification. For example the quenched and scrubbed ethylene stream is compressed to a pressure from about 0.1 MPa to about 3 MPa, caustic washed, and dried. The dried ethylene stream may be fed to a demethanizer to remove C 1 hydrocarbons and finally, to a 'C2' splitter where an ethylene product is produced as an overhead stream. The ethylene produced after purification may be again compressed to the operating pressure of the eventual downstream process.

Efforts have been made to produce ethylene by renewable ethanol via dehydration at elevated pressure such that its downstream use avoids subsequent compression for the overall process to become more economical.

An example of development of catalytic dehydration reaction capable of accepting aqueous ethanol as its feedstock for the formation of olefins under superatmospheric condition (>1 atm) can be found in WO 2011/037681. In WO'681, the catalytic process continuously flows through a reaction zone a liquid phase containing an aliphatic C2-C6 alcohol to contact a non-volatile acid catalyst at a reaction temperature and pressure to at least partially convert the aliphatic C2-C6 alcohol in the liquid phase to its corresponding olefin. The reaction pressure is greater than atmospheric pressure and the reaction temperature is above the boiling point of the olefin at reaction pressure, but below the critical temperature of the alcohol, and the olefin product is substantially in the gaseous phase. A non-volatile acid catalyst, preferably a solid acid catalyst is used in this process. Solid acid catalysts useful in the invention preferably include isomorphously substituted aluminium phosphate (A1PO) catalysts such as disclosed in WO 2010/085708 (incorporated herein by reference). Unsubstituted AlPO's (see U.S. Pat. No. 3,915, 893) and other strong solid acids such as SAPO's, silicalites, and zeolites may also be employed. Zeolite ZSM-5 (see U.S. Pat. No. 3,702,886 and U.S. Pat. No. 4,100,262) is of particular interest because it has been shown to convert ethanol to ethylene in the vapor phase. Other non-volatile acids known in the art, such as sulphuric acid, p-toluenesulfonic acid, methanesulfonic acid, heteropoly acids or phosphoric acid may also be used in this process.

As a result of the dehydration of ethanol, it should be noted that by-products such as alkanes (e.g., methane, ethane), aldehydes (e.g, acetaldehyde), ketones, ethers (e.g., diethylether), and oligomers may be formed. Some byproducts can be formed by coupling of alkyl fragments e.g., acid catalysed olefin oligomerisation, such as: 2 propylene → hexene. Some by-products may be formed by alcohol dehydrogenation, e.g., ethanol → acetaldehyde + H₂ (Y.Matsumura, K.Hashimoto, and S.Yoshida, J. Catalysis 1989, vol. 117, pp. 135-143).The state of the hydrogen liberated may not be as free hydrogen but as chemisorbed hydrogen. Of particular relevance is the transfer hydrogenation reaction e.g., ethylene + H2 → ethane; and 2 ethanol → acetaldehyde + ethane + water. The formation of same carbon number alkane is known to add significantly to the complexity and cost of producing purified olefins for polymer manufacture.

The ethanol-to-ethylene processes described above present the disadvantages of requiring several difficult steps of fractionation to obtain at least one purified fraction containing ethylene.

There is a need for an improved and/or alternative process for the production of ethylene derivatives (particularly 1,2-dichloroethane) from ethanol. A solution to these and other disadvantages is provided by the present invention, which relates specifically to a process to form ethylene derivative(s) from ethylene obtained via the dehydration of ethanol, such process not requiring costly separation to remove C1 and C2 alkanes.

There is also a need to provide a process for the manufacture of at least one ethylene derivative compound, in particular of at least DCE, using ethylene with a purity of less than 99.8 % which does not present the disadvantages of the above-mentioned processes using ethylene having a purity of less than 99.8 % and which allows the use of an alcohol feedstock which is derived from renewable resources.

The solution to these and other disadvantages is provided by the present invention, which relates specifically to a process which utilizes a selective purification scheme to generate at least two ethylene-containing feedstreams derived from dehydration of bioethanol to form at least one ethylene derivative. The purification scheme is selective in that it comprises at least one purification step to generate one of the two feedstreams which is not performed to generate the other feedstream.

### Summary

To this effect, one embodiment of the present invention relates to a process for the manufacture of at least one ethylene derivative compound starting from a biomass-derived alcohol, particularly a biomass-derived ethanol also termed 'bioethanol' or 'hydrous ethanol'.

This process for the manufacture of at least one ethylene derivative from bioethanol, comprises the following steps:
a) subjecting a feedstock comprising biomass-derived ethanol to dehydration promoting conditions to produce an olefins stream comprising ethylene;
b) separating the olefins stream to form at least one fraction containing most of the ethylene (fraction A), said fraction A further comprising oxygenates and water;
c) optionally compressing at least a portion of fraction A to form a compressed stream A1;
d) dividing said fraction A or said compressed stream A 1 into a first slipstream B1 and a second slipstream B2;
e) passing at least a portion of the second slipstream B2 through a drying unit to form a dried ethylene-containing stream;
f) performing an oxygenate removal step selected from the group consisting of:
   f1) removing at least a portion of oxygenates from said second slipstream B2 before drying step (e);
   f2) removing at least a portion of oxygenates from the dried ethylene-containing stream after drying step (e); and
   f3) removing at least a portion of oxygenates from said slipstream B2 before drying step (e) and removing another portion of oxygenates from the dried ethylene-containing stream after drying step (e),
   wherein steps (e) and (f) are effective in generating an ethylene-containing feedstream C2, which is leaner in water and oxygenates than the second slipstream B2 formed in step (d);
g) conveying the first slipstream B 1 or a portion thereof to a first reactor to convert ethylene to at least one first ethylene derivative and to generate a first product stream P1 comprising said formed first ethylene derivative; and
h) conveying the ethylene-containing feedstream C2 or a portion thereof to a second reactor to convert ethylene to at least one second ethylene derivative and to generate a second product stream P2 comprising said formed second ethylene derivative, said second ethylene derivative being optionally the same as the first ethylene derivative formed in step (g).

In some embodiments, the operating pressure in the first and second reactors are different; preferably the operating pressure in the second reactor is greater than the operating pressure in the first reactor.

In preferred embodiments, the process includes the main compression step (c) being carried out upstream of the split point from which slipstreams B1 and B2 are formed.

In alternate embodiments, the process omits the main compression step (c) upstream of the split point from which slipstreams B1 and B2 are formed. In such instance, the process may include a secondary compression step (i) being carried out downstream of the split point from which slipstreams B1 and B2 are formed.

In a first variant, the process comprises performing the oxygenates removal step (f1). In such instance, the process may optionally comprise: (i1) carrying out a secondary compression on the slipstream (B2) before the oxygenates removal step.

In a second variant, the process comprises performing the oxygenates removal step (f2) or step (f3). In such instances, the oxygenates removal step performed after the drying step (e) may include a fractionation. In such instance, the process may optionally comprise: (i2) carrying out a secondary compression step on the second slipstream B2 before fractionation and preferably before drying step (e).

In a third variant, the process comprises performing the oxygenates removal step (f3). In such instance, the oxygenates removal step carried out after drying step (e) may include a fractionation, and the oxygenates removal step carried out before drying step (e) may exclude a fractionation. In such instance, the process may optionally comprise: (i3) carrying out a secondary compression step on the second slipstream B2 before the drying step (e) and before the oxygenates removal step which includes a fractionation. Such secondary compression in step (i3) may be carried out before the oxygenates removal step which is performed before drying step (e), or may be carried out after an oxygenates removal step which does not include a fractionation and before drying step (e).

In some embodiments, the at least one ethylene derivative compound formed in steps (g) and (h) may comprise or may be 1,2-dichloroethane (DCE). The first and second reactors may employ the same reaction conditions, but preferably employ different reaction conditions. The first reactor preferably employs oxychlorination reaction conditions, while the second reactor preferably employs chlorination reaction conditions, both of these reactors being effective in converting ethylene to DCE.

In some embodiments, the product stream P2 comprises unconverted ethylene, and at least a portion of the product stream P2 is directed to the first reaction in order for at least a part of unconverted ethylene originating from said portion of product stream P2 to get converted to the first ethylene derivative.

In some embodiments, the process may further comprise performing an upstream desulfurization step (j') which comprises subjecting the fraction A or compressed stream A 1 to desulfurization before forming the slipstreams B1 and B2. The desulfurization technique used for upstream desulfurization step (j') may be selected from the group consisting of an alkali wash, a reaction with a peroxide (e.g., H2O2), an activated carbon adsorption, an alkali metal hydride adsorption, and combinations of two or more thereof.

In alternate or additional embodiments, the process may further comprise performing a desulfurization step (j) after slipstreams B1 and B2 are formed, wherein the desulfurization step (j) comprises at least one of the following steps:
(j1) subjecting the slipstream B2 or a portion thereof to a desulfurization technique selected from the group consisting of an alkali wash, an activated carbon adsorption, an alkali metal hydride adsorption, and combinations of two or more thereof, said step (j1) being carried out before the drying step (e) and before the oxygenates removal step (f);
(j2) subjecting the slipstream B2 or a portion thereof to a desulfurization technique selected from the group consisting of an alkali wash, an activated carbon adsorption, an alkali metal hydride adsorption, and combinations of two or more thereof, said step (j2) being carried out in between the drying step (e) and the oxygenates removal step (f);
(j3) subjecting the slipstream B2 or a portion thereof during the oxygenates removal step (f1) to a desulfurization technique selected from the group consisting of an alkali wash, an alkali metal hydride adsorption, and combination thereof;
(j4) subjecting the ethylene-containing feedstream C2 resulting from step (e) and step (f) to a desulfurization technique which includes an activated carbon adsorption;
(j5) subjecting the slipstream B1 to said desulfurization technique selected from the group consisting of an alkali wash, an activated carbon adsorption, an alkali metal hydride adsorption, and combinations of two or more thereof; and any combinations of two or more thereof.

In alternate or additional embodiments, the process may further comprise :
(k) passing at least a portion of the first product stream P1, at least a portion of the second product stream P2, or any combination thereof through a third reactor under suitable conditions to generate at least one first compound derived therefrom which is different from the ethylene derivative(s) formed in steps (g) and (h). The process may comprise: mixing at least a portion of the product stream P1 formed in step (g) and at least a portion of the product stream P2 formed in step (h), and passing such mixture to the third reactor. The at least one first compound derived therefrom exiting the third reactor is preferably vinyl chloride, when the at least one ethylene derivative compound formed in steps (g) and (h) is 1,2-dichloroethane.

In alternate or additional embodiments when the at least one ethylene derivative compound formed in steps (g) and (h) is 1,2-dichloroethane, the process may further comprise :
(k) passing at least a portion of the 1,2-dichloroethane formed in step (g), at least a portion of the 1,2-dichloroethane formed in step (h), or any combination thereof through a third reactor under suitable 1,2-dichloroethane cracking conditions to generate vinyl chloride. The process may comprise:
   mixing at least a portion of the product stream P1 comprising 1,2-dichloroethane formed in step (g) and at least a portion of the product stream P2 comprising 1,2-dichloroethane formed in step (h), and subjecting such mixture to said 1,2-dichloroethane cracking conditions in the third reactor.

In embodiments when the at least one ethylene derivative compound formed in steps (g) and (h) is 1,2-dichloroethane, and the process includes step (k), the process may further comprise:
(1) polymerizing the vinyl chloride produced in step (k) to produce polyvinyl chloride as a second compound derived therefrom.

The foregoing has outlined rather broadly the features and technical advantages of the present invention in order that the detailed description of the invention that follows may be better understood. Additional features and advantages of the invention will be described hereinafter that form the subject of the claims of the invention. It should be appreciated by those skilled in the art that the conception and the specific embodiments disclosed may be readily utilized as a basis for modifying or designing other structures for carrying out the same purposes of the present invention. It should also be realized by those skilled in the art that such equivalent constructions do not depart from the spirit and scope of the invention.

### Brief description of the drawings

For a detailed description of the preferred embodiments of the invention, reference will now be made to the accompanying drawings in which:

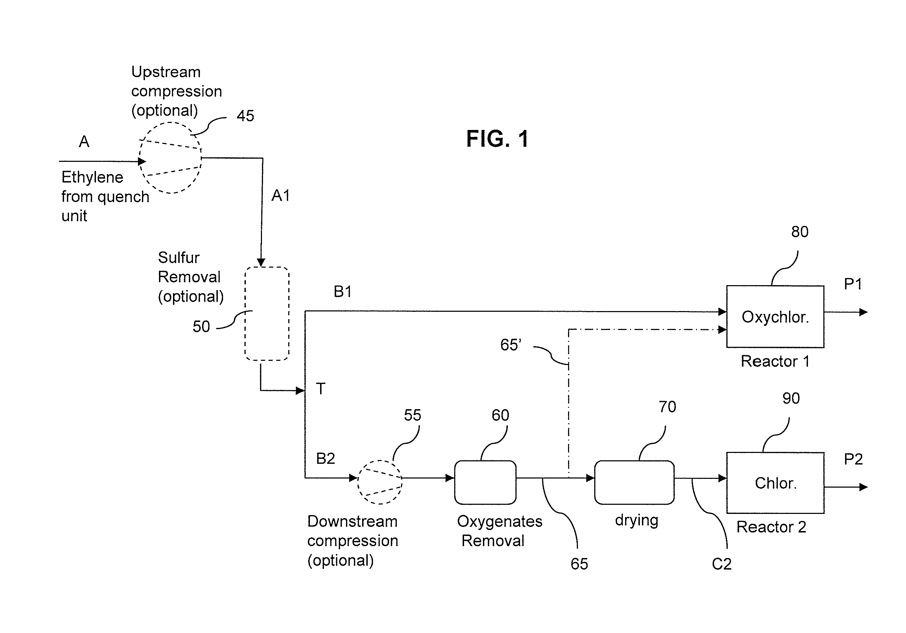

FIG. 1 illustrates a process flow diagram according to a first embodiment of the process for the conversion of ethylene slipstreams B1 and B2 to produce product streams P1 and P2 in separate oxychlorination and chlorination reactors, wherein the slipstream B2 is subjected to oxygenates removal followed by drying before chlorination, and wherein this process may further have an optional upstream desulfurization step carried out before the split point T from which slipstreams B1 and B2 are formed;

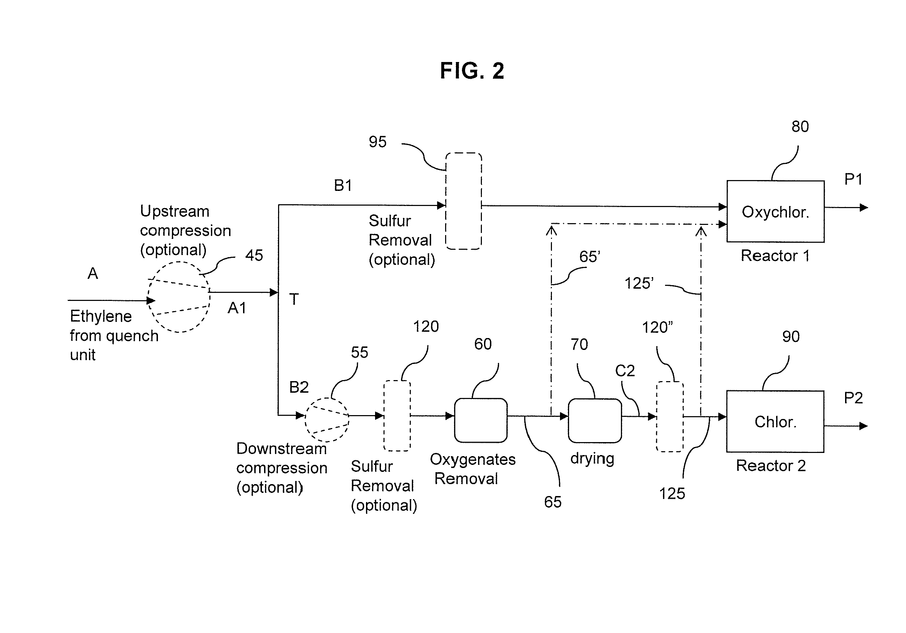

FIG. 2 illustrates a process flow diagram according to a second embodiment of the process which is similar than FIG. 1 except that, instead of having an optional upstream desulfurization step, this process may include an optional downstream desulfurization step carried out before and/or after the drying step and/or may have an optional downstream desulfurization step carried out before oxychlorination or before chlorination;

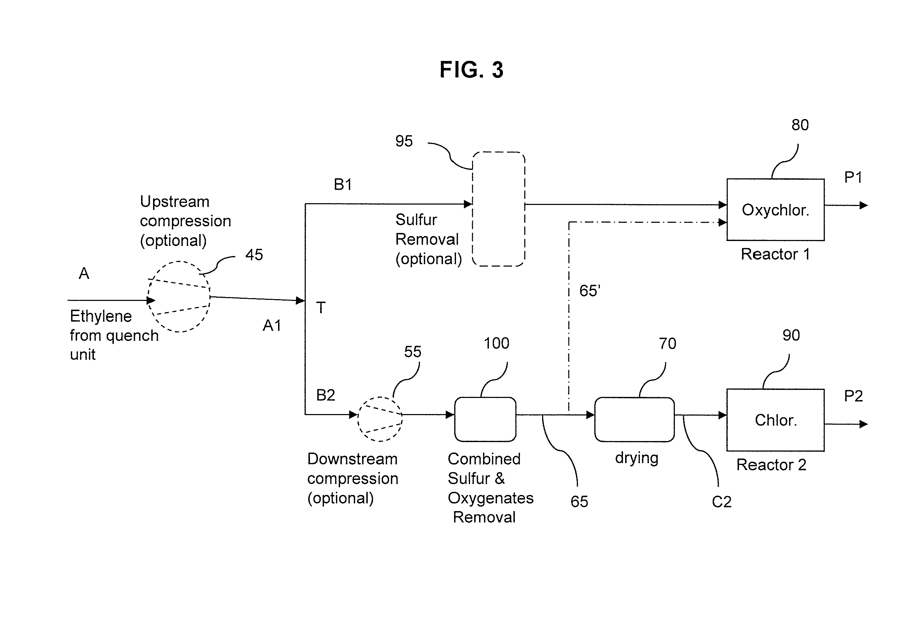

FIG. 3 illustrates a process flow diagram according to a third embodiment of the process for the conversion of ethylene slipstreams B1 and B2 to produce product streams P1 and P2 in separate oxychlorination and chlorination reactors, wherein the slipstream B2 is subjected to a combined sulfur and oxygenates removal step followed by drying before chlorination, and wherein this process may further have an optional downstream desulfurization step separately carried out on at least a portion of the slipstreams B 1 before oxychlorination;

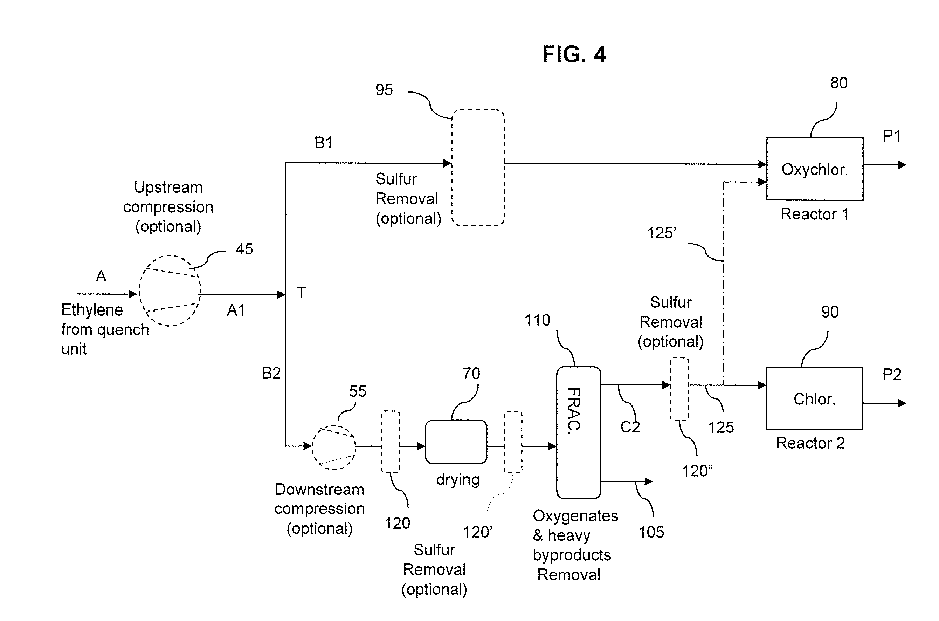

FIG. 4 illustrates a process flow diagram according to a fourth embodiment of the process for the conversion of ethylene slipstreams B1 and B2 to produce product streams P1 and P2 in separate oxychlorination and chlorination reactors, wherein the slipstream B2 is subjected to drying followed by a fractionation which serves as an oxygenates removal step to form the feedstream C2 which is directed to chlorination, and wherein this process may further have one or more optional downstream desulfurization steps separately carried out on at least a portion of the slipstream B2 before chlorination and/or at least a portion of the slipstream B 1 before oxychlorination;

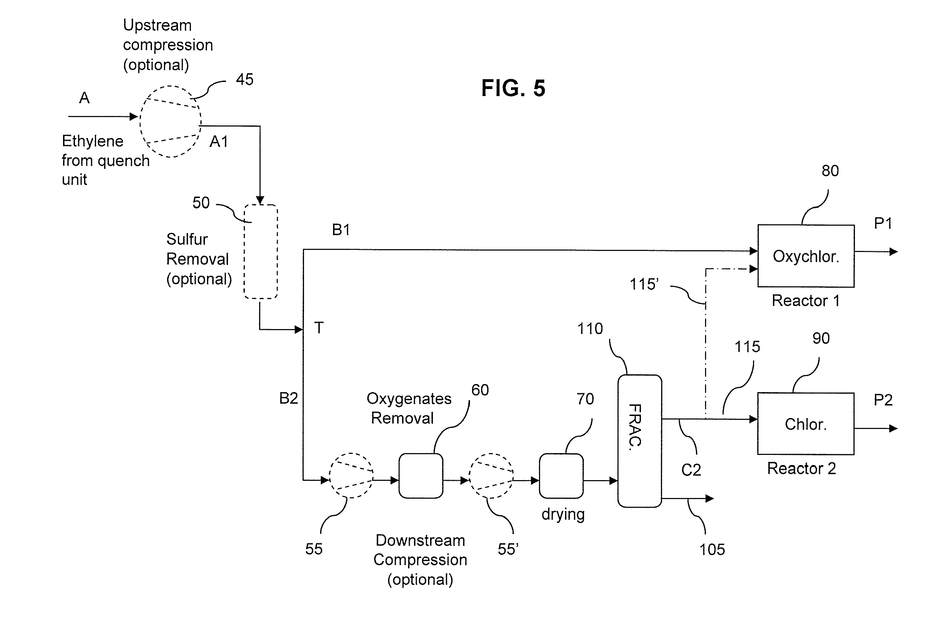

FIG. 5 illustrates a process flow diagram according to a fifth embodiment of the process for the conversion of ethylene slipstreams B1 and B2 to produce product streams P1 and P2 in separate oxychlorination and chlorination reactors, wherein the slipstream B2 is first subjected to an oxygenates removal, second is subjected to a drying step, and third is sent to a fractionation step (which can serve as an additional oxygenates removal step) to form the feedstream C2 which is directed to chlorination;

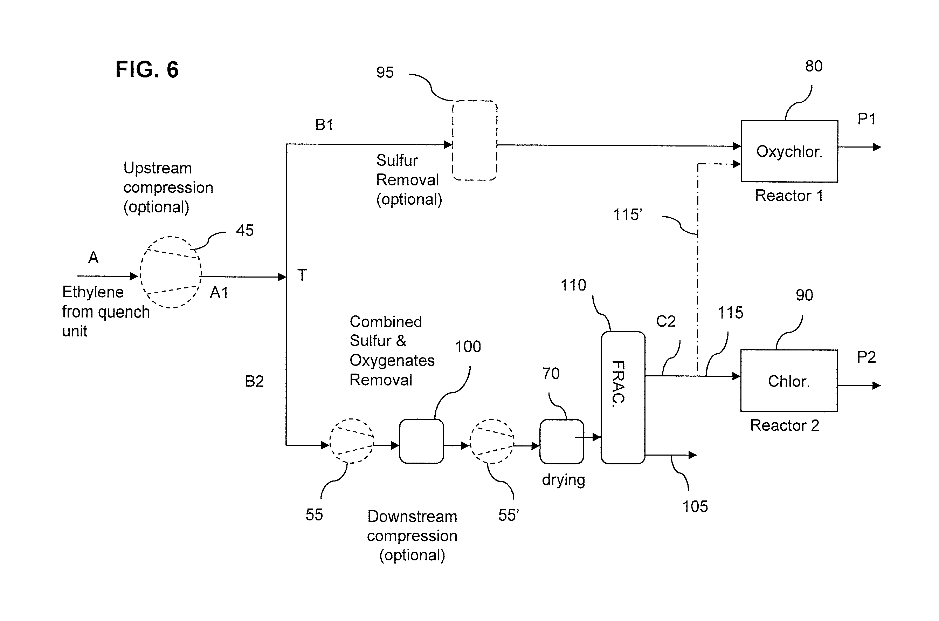

FIG. 6 illustrates a process flow diagram according to a sixth embodiment of the process which is similar than FIG. 3 except that this process further includes a fractionation step being carried out after the oxygenates removal and drying steps and an optional downstream compression step performed before drying and fractionation;

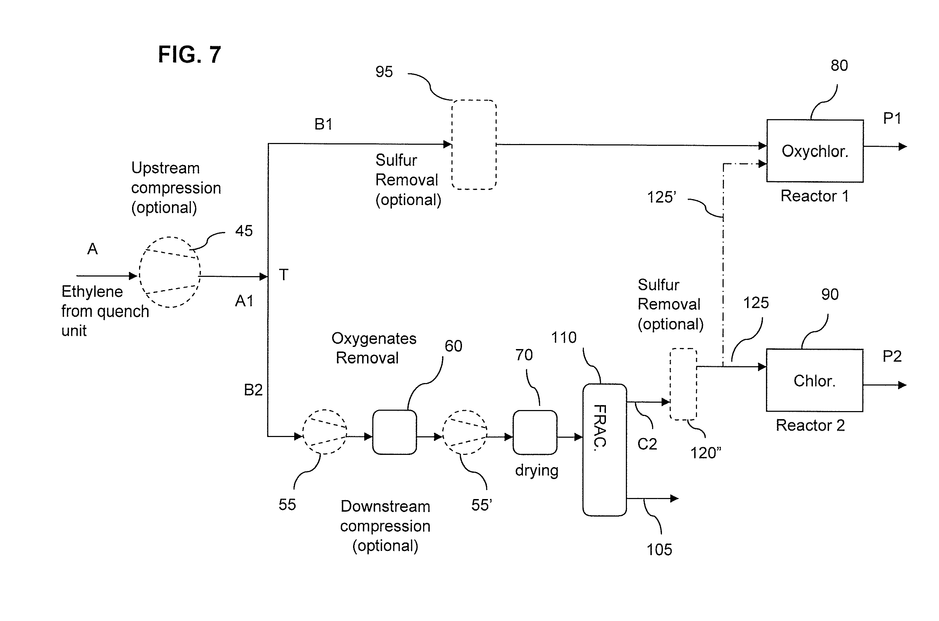

FIG. 7 illustrates a process flow diagram according to a seventh embodiment of the process which is similar than FIG. 5 except that, instead of having an optional upstream desulfurization step, this process may include an optional downstream desulfurization step carried out on the feedstream C2 before chlorination and/or may have an optional downstream desulfurization step carried out on the slipstream B 1 before oxychlorination; and

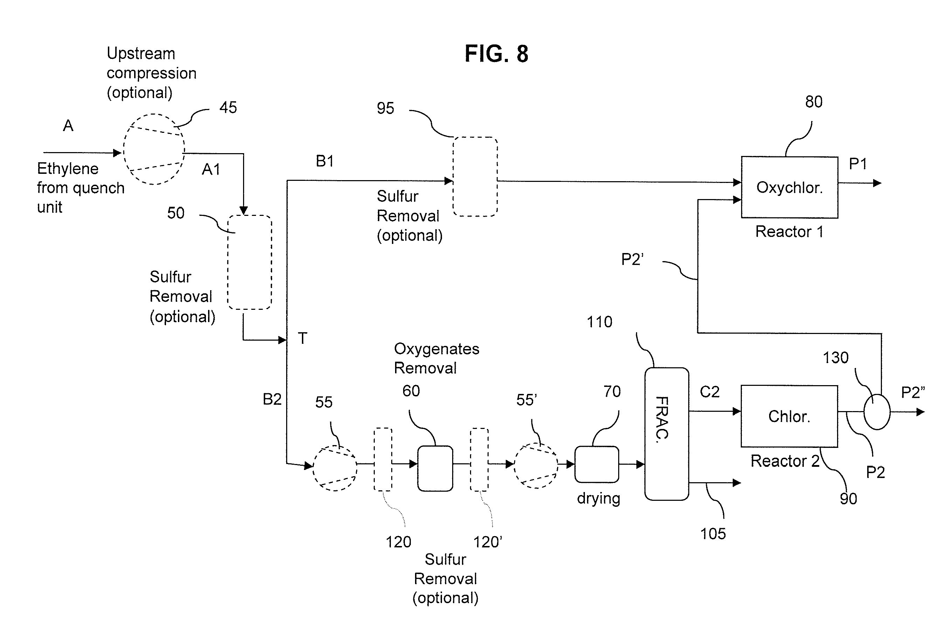

FIG. 8 illustrates a process flow diagram according to an eighth embodiment of the process for the conversion of ethylene slipstreams B1 and B2 to produce product streams P1 and P2 in separate oxychlorination and chlorination reactors, wherein the slipstream B2 is subjected to an oxygenates removal step, to a drying step, then to a fractionation (which can serve as an additional oxygenates removal step) to form an ethylene-containing light fraction which is directed to chlorination, and wherein at least a portion of the resulting product stream P2 exiting the chlorination reactor is sent to the oxychlorination reactor.

### Definitions and nomenclatures

The expression "at least one ethylene derivative compound" is understood to mean, for the purpose of the present invention, that one or more than one ethylene derivative compounds may be manufactured by the process according to the present invention.

The expression "ethylene derivative compound manufactured directly starting with ethylene", used hereafter in the singular or in the plural, is understood to mean, for the purpose of the present invention, any compound manufactured directly from ethylene.

The expression "compound derived therefrom", used hereafter in the singular or in the plural, is understood to mean, for the purpose of the present invention, any compound manufactured from one compound itself manufactured from ethylene as well as any compound derived therefrom.

The expression "ethylene derivative compound", used hereafter in the singular or in the plural, is understood to mean, for the purpose of the present invention, any ethylene derivative compound manufactured directly starting with ethylene as well as any compound derived therefrom.

As examples of such ethylene derivative compounds manufactured directly starting with ethylene, may be cited among others, ethylene oxide, linear alphaolefins, linear primary alcohols, homopolymers and copolymers of ethylene, ethylbenzene, vinyl acetate, acetaldehyde, ethyl alcohol, propionaldehyde, and DCE.

As examples of such compound derived therefrom, may be cited among others,
- glycols and ethers manufactured from ethylene oxide,
- styrene manufactured from ethylbenzene and polymers of styrene derived from styrene,
- vinyl chloride (VC) manufactured from DCE,
- vinylidene chloride, fluorinated hydrocarbons and polyvinyl chloride (PVC) derived from VC and fluorinated polymers derived from fluorinated hydrocarbons, as well as
- polyvinylidene chloride and fluorinated hydrocarbons (and fluorinated polymers) derived from vinylidene chloride.

The process according to the invention is a process starting from a biomass-derived ethanol.

The expressions "a biomass-derived ethanol" or "bioethanol", used hereafter in the singular, are understood to mean, for the purpose of the present invention, one alcohol-containing stream containing ethanol, which is produced by conversion of biomass (e.g., sugarcane, corn, switchgrass, wheat, and/or agricultural waste) and purified so as to contain at least 85% ethanol. Such bioethanol may contain up to 15% water. Examples of oxygen-containing impurities in bioethanol include methanol, isopropanol, n-propanol, other higher molecular weight alcohols, and acetaldehyde.

The expression "undesirable component" is understood to mean, for the purpose of the present invention, a component present in an ethylene stream which is to be at least partially removed if it is harmful for at least one of the steps of the process (i.e., it negatively impacts the main reaction taking place in the first and/or second reactor).

The expression "at least partially removed" is understood to mean, for the purpose of the present invention, that advantageously at least 15 %, preferably at least 20 %, more preferably at least 25 % of the quantity of each undesirable component present in the ethylene-containing stream or fraction is removed. Advantageously, at most 90 % of the quantity of such each undesirable component is removed.

The expression "substantially removed" is understood to mean, for the purpose of the present invention, that advantageously at least 95 %, preferably at least 98 %, more preferably at least 99 % of the quantity of each undesirable component present in the ethylene-containing stream or fraction is removed.

The terms 'upstream' and 'downstream' in front of 'desulfurization' and/or 'compression unit' refer to the positioning of this unit with respect to the split point 'T' from which slipstreams B1 and B2 are divided. For example, an 'upstream' desulfurization unit is positioned before this split point T and can receive the fraction A, any portion thereof, or the compressed stream A1, or any portion thereof, whereas a 'downstream' desulfurization unit is positioned after this split point T and can receive any stream created after such point T (e.g., either of the slipstreams B1 and B2 or any portion thereof.

### Detailed description

The present invention particularly relates to a process for the production of an ethylene derivative from an ethylene-containing fraction A which is obtained from the dehydration of bioethanol.

One objective of the present invention relates to a process to make an ethylene derivative(s) in two or more reactors operating under different reaction conditions, each of which utilizing an ethylene-containing stream obtained from dehydration of bioethanol.

Another objective of the present invention relates to a selective purification method to provide suitable ethylene-containing feedstreams to different reactors where specific step(s) to remove one or more undesirable components (also called 'impurities') present in small quantity in a raw ethylene material derived from dehydration of bioethanol are implemented when the operation of at least one of such first and second reactors may be negatively impacted by at least some of these undesirable components. These undesirable components generally originate from the bioethanol source, but may have been transformed under dehydration conditions when ethanol is converted to ethylene from an initial form present in the bioethanol stream to a modified form present in the resulting olefins stream.

### Bioethanol

The process according to the invention preferably uses an impure ethanol feedstock. The impure ethanol feedstock may be anhydrous or hydrous, preferably hydrous. The ethanol feedstock preferably comprises biomass-derived ethanol. The biomass-derived ethanol may be anhydrous or hydrous, preferably hydrous bioethanol. The ethanol feedstock most preferably consists essentially of hydrous bioethanol.

The hydrous bioethanol may have an ethanol content of at least 90%, preferably between 92 and 95%, more preferably between 92.5 and 94%. The hydrous bioethanol may have a water content of from 3 to 8% water, preferably between 5 and 7%. The density of this hydrous bioethanol may be between 807 and 811 kg/m³.

Impurities in the hydrous bioethanol may include oxygen-containing compounds, also termed 'oxygenates':
- aldehydes such as acetaldehyde, crotonaldehyde;
- other alcohols, such as methanol, isopropanol, n-propanol, n-butanol, isobutanol, isoamylic alcohol;
- alkanes, such as n-hexane, cyclohexane;
- ketones, such as acetone; and/or
- esters, such as ethyl acetate.

The impurities in such hydrous bioethanol may include sulfur-containing compounds:
- inorganic sulfur, such as sulfate which can be added for pH control during fermentation or sulfite which can be utilized for treatment of biomass juice for the production of refined sugar;
- symmetrical or unsymmetrical C₂-10-dialkyl sulfides, particularly C₂-6-dialkyl sulfides (such as diethyl sulfide, di-n-propyl sulfide, diisopropyl sulfide), very particularly dimethyl sulfide;
- symmetrical or unsymmetrical C₂-10-dialkyl disulfides, particularly C₂-6-dialkyl disulfides, such as dimethyl disulfide;
- symmetrical or unsymmetrical C₂-10-dialkyl sulfoxides, such as dimethyl sulfoxide, diethyl sulfoxide, dipropyl sulfoxide;
- C₁-10-alkyl mercaptans, such as methyl mercaptan, ethyl mercaptan, n-propyl mercaptan;
- 3-methylthio-1-propanol; and/or
- S-containing amino acids, such as methionine and S-methylmethionine.

Other impurities in such hydrous bioethanol may include compounds containing other heteroatoms chosen from Fe, Na, or Cl.

Any of the above-mentioned impurities can be designated as undesirable components.

### Step (a): Dehydration

Dehydration may employ a vapor-phase or liquid-phase reaction which takes place in a dehydration reactor.

When the dehydration uses a vapor-phase reaction, the ethanol feedstock may be vaporized (for example by the use of steam) and fed to a dehydration reactor.

The dehydration reactor may be operated under isothermal or adiabatic conditions, preferably adiabatic conditions.

The dehydration reactor or reaction zone may contain a fluidized bed or a fixed bed.

Since the dehydration reaction is endothermic, there needs to be an input of heat provided to the reactor. For a single bed adiabatic reactor the overall endothermic reaction if allowed to go to thermodynamic equilibrium could result in a theoretical temperature drop of 180°C. Heat management can be addressed by reactor design. Suitable reactor designs include those capable of handling heat fluxes such as fixed bed, fluidized bed, multi-tubular and multiple fixed bed reactors with inter-stage heating zones. Optionally the heat management can be improved by injecting preheated fresh alcohol feed at several points in the reactor bed. The ethanol feedstock may be preheated for example in a furnace to a temperature above the reaction temperature (e.g., at about 400 °C), in order to provide an additional source of heat. A portion of an ethanol recycle stream also can be added at several points along the reactor with additional heating; alternatively the main portion of such recycle stream may be added to the front end of the reactor. In embodiments when a plurality of dehydration reaction zones operates in series, the gas effluent exiting a reaction zone is heated prior to entering the subsequent reaction zone; such intermediate heating can be done by passing the gas effluent in the same furnace through which the fresh ethanol feedstock is preheated (albeit in separate flow paths).

The dehydration reactor may comprise more than one dehydration reaction zone. Two or more dehydration reaction zones may be operated in series with inter-stage heating zones sandwiched between two consecutive dehydration reaction zones to allow intermediary heat input. Alternatively, the two or more dehydration reaction zones may be operated in parallel so as to allow regeneration of dehydration catalyst contained herein. The different dehydration reaction zones may be housed in separate vessels or in a single vessel.

An example of an isothermal reactor includes isothermal tubular reaction zones inside which the dehydration reaction takes place and outside which a heating medium circulates to provide heat of dehydration reaction.

An example of an adiabatic reactor includes a reactor inside which two or more dehydration reaction zones are located. The adiabatic dehydration reactor preferably comprises a plurality of dehydration reaction zones operated in series (for several consecutive dehydration stages) in form of fixed beds and one or more inter-stages zones sandwiched between two consecutives fixed beds where additional heat may be provided. As an effluent gas exits a first dehydration reaction zone to be directed to a second dehydration reaction zone, it may be heated *in situ* in the inter-stage zone positioned immediately downstream of the first dehydration reaction zone and immediately upstream of a second dehydration reaction zone or may be heated *ex situ* by passing through an external heating zone (e.g., furnace) which may be different or preferably the same as the one through which the ethanol feedstock is preheated, and such heated effluent gas serves as feedstock to the second dehydration reaction zone.

Ethylene production from ethanol dehydration is a widely known process. In this process, ethanol is converted into ethylene by means of a catalytic reaction at a temperature of at least 180°C, preferably of at least 300°C.

In preferred embodiments, step (a) comprises contacting the feedstock comprising the biomass-derived ethanol with a dehydration catalyst at a temperature of at least 300°C, more preferably from 310 to 450°C.

In other embodiments, step (a) comprises contacting the feedstock comprising the biomass-derived ethanol with a dehydration catalyst at a temperature of at least 180°C but less than 300°C.

In preferred embodiments, step (a) comprises contacting the feedstock comprising the biomass-derived ethanol with a dehydration catalyst at a pressure from 0.5 atm to about 25 atm absolute (from 50 kPa to 2.5 MPa), preferably from 1 to 5 atm (from 101 to 505 kPa), most preferably from 1.1 to 3 atm (from 110 to 303 kPa). Alternate embodiments may comprise a dehydration operating pressure of from 2 to 25 atm (from 202 to 2525 kPa), or from 2 to 20 atm (from 202 to 2020 kPa), or even from 2 to 15 atm (from 202 kPa to 1515 kPa).

The dehydration step (a) is carried out with a dehydration catalyst comprising an acidic component. A large variety of acid catalysts can be used for the dehydration step (a), but the most commonly used solid types include pure or doped high specific surface area gamma-alumina (γ -Al₂O₃), titania/ y - alumina, magnesium oxide, H-mordenites, silica-aluminate such zeolites (e.g., H-ZSM 5), silica/titania, silicoaluminophosphates ('SAPO' catalysts), and silica-alumina. Other catalysts may be used for liquid-phase dehydration. Preferred examples are solid acid catalysts including isomorphously substituted aluminum phosphate (AlPO) catalysts such as disclosed in WO 2010/085708 (incorporated herein by reference). Unsubstituted AlPO's (see US 3,915,893 incorporated herein by reference) and other strong solid acids such as SAPO's, silicalites, and zeolites (e.g., Zeolite ZSM-5) can be employed. Other non-volatile acids, such as sulfuric acid, p-toluenesulfonic acid, methanesulfonic acid, heteropoly acids or phosphoric acid may also be used in the dehydration step.

Further details about this dehydration step (a) can be found in the following study: "Ethylene from Ethanol", Harold W. Scheeline and Ryoji Itoh, PEP Review 79-3-4, January 1980, SRI International, and also in patents No. U.S. Pat. Nos. 4,232,179; 4,234,752; 4,396,789; 4,398,050; 4,423,270; 4,529,827; 4,727,214; 5,475,183; and GB 2094829, as well as in the patent application WO 2004/078336, these applications being incorporated by reference to the extent of their teachings concerning dehydration operating conditions and catalyst compositions. For example, U.S. Pat. No. 5,475,183 describes a process for producing light olefins by dehydrating lower alcohols having 2-4 carbon atoms on a alumina catalyst in the vapor phase. The typical reaction conditions given in the examples are 300-400°C at 9 to 19 bars with reported olefin selectivities between 65 and 97%. U.S. Pat. No. 4,232,179 describes how ethanol can be dehydrated in adiabatic reactors. The examples, with silica /alumina, and alumina show that the ethane content in the ethylene product is above 923-100,000 ppm weight on ethylene. This would be unacceptable for polyethylene production without additional purification. U.S. Pat. No. 4,398,050 describes the synthesis of a mixed alcohol stream and purification to give a mixture of ethanol and propanol which is subsequently dehydrated at 0.5-1 bar, 350-500°C (example 1). The primary claim mentions the removal of methanol prior to dehydration, but not the removal of C4 and higher alcohols. U.S. Pat. No. 4,423,270 describes the atmospheric pressure vapor phase dehydration of ethanol over a supported phosphoric acid catalyst with additional water and an alkyl substituted phosphoric acid. The reaction temperatures employed are between 300-400 °C and the experiments were conducted at atmospheric pressure in a glass tube. The reported yields of ethylene ranged from 88-101%, albeit there are no details on by-product formation. U.S. Pat. No. 4,727,214 describes the dehydration of ethanol over a crystalline aluminosilicate zeolite. The conditions include a pressure between 1 and 10 bars and a temperature between 126°C and 526°C. Details of by-product formation are supplied to one decimal place and a selectivity to ethylene of 100% is reported. GB 2094829 describes how ethylene can be produced in a plurality of vapor phase adiabatic reactors with parts of the liquid products containing unconverted alcohol being recycled. The reaction conditions are described as the feed charge is at 400-520°C and 20-40 bars. The outlet product is kept at least at 19 bars prior to being cryogenically purified.

It should be noted that during the dehydration of ethanol, by-products are generally formed. These can be formed by coupling of alkyl fragments, e.g., acid catalyzed olefin oligomerization, such as 2 propylene → hexene; or by alcohol dehydrogenation, such as ethanol → acetaldehyde + H₂; or transfer hydrogenation reaction, such as ethylene + H₂ → ethane. The formation of same carbon number alkanes is known to add significantly cost in separation in order to produce polyethylene-grade ethylene. However, alkanes presence in the ethylene stream does not necessarily require their removal, as some processes which produce ethylene derivatives may not be negatively impacted by the presence of alkanes in ethylene. For example, it has been found that the presence of alkanes (e.g., ethane) in the ethylene stream does not poison or impede an oxychlorination reaction. Thus to make DCE as one ethylene derivative via oxychlorination, there is no need to separate ethane from ethylene by way of very expensive fractionation. The same is true for oxygenates, as it has been found that the oxychlorination reaction is not impacted by the presence of oxygenates in the ethylene product formed via dehydration.

However oxygenates (e.g., aldehyde and alcohols) in the ethylene feedstream can negatively impact a chlorination reaction, and as such at least a portion of these oxygenates need to be removed from the portion of the ethylene stream which is fed to the chlorination reactor.

### Step (b): Separation

The process comprises separating the olefin stream comprising ethylene which is formed during the dehydration step (a) to form a fraction containing most of the ethylene (fraction A). The separation step preferably includes a quench device, generally a column.

The quench device is generally fed from vapor effluent of a waste heat boiler which receives the hot dehydration reactor effluent. As used herein, "quench device" is a device for removing some components of the reactor effluent by establishing a sufficient quantity of liquid quench medium in contact with a gaseous dehydration reactor which condenses at least a portion of some components from the reactor. A "quench medium" is defined as a liquid that contacts the dehydration reactor effluent in a quench device. Examples of a quench device in an oxygenate-to-olefin product stream can be found in U.S. Pat. No. 6,121,504 (direct product quench) and U.S. Pat. No. 7,005,555 (removal of oxygenate(s) including carbon dioxide in the bottoms of the quench stream and recycle of the oxygenate(s) back to the reactor), each fully incorporated herein by reference.

The quench device in one embodiment may have a single stage or multiple stages. The multiple-stages quench device of a particular embodiment preferably has two to four stages, more preferably two to three stages. The quench device is optionally in single or multiple housings or towers.

The quench device typically includes internal elements in a contact zone to facilitate intimate contacting of the quench medium with the reactor effluent or portions thereof. Contact zone is defined as the zone of the quench device where the dehydration reactor effluent comes in contact with the quench medium. Internal elements include liquid distributors and contacting devices such as baffles, trays, random packing, or structured packing.

Generally, the hot olefin stream exiting the dehydration reactor is directed to the quench device. The hot olefin stream is preferably quenched with a quench medium in the quench device to produce a quench bottoms stream as well as a quenched olefin stream which provides the ethylene-containing fraction A after such separation step (b).

As used herein, the term, "quench bottoms stream" refers to the portion of the reactor and quench medium that is liquid under quench conditions and includes all streams that contain the condensed portion of the olefins stream and fractions of the condensed olefins stream. The term "quenched olefins stream" refers to the portion of the reactor olefins stream that is gaseous after at least one step of quenching.

The quenched olefin stream or "ethylene-containing fraction A" is predominantly gaseous and, in preferred embodiments, leaves the quench device. The quench bottoms stream may contains a majority of catalyst fines which may be present in the dehydration reactor. The quench bottoms stream is withdrawn optionally for additional processing. In one embodiment, a quench medium is supplied to the quench device. According to one embodiment, the quench medium is an aqueous quench medium. In another embodiment, the quench medium is an aqueous quench medium that is pH adjusted by adding alkaline, preferably caustic. In still another embodiment, the pH is adjusted by adding ammonium or amines.

According to an embodiment, the pH of the quench medium as it enters the quench device is greater than 7. Preferably, the pH of the quench medium as it enters the quench device ranges from about 7.1 to about 11.5, more preferably ranges from about 7.5 to about 11, even more preferably ranges from about 8 to about 10 at the time the quench medium enters the quench device. Most preferably, the pH of the quench medium as it enters the quench device is about 9.

The quench device bottoms temperature is from about 180°F. (82°C.) to about 300°F. (149°C.); preferably from about 180°F. (82°C.) to about 250°F. (121°C.); more preferably about 200°F. (93°C.). The temperature of the quench medium is from about 60°F. (15°C.) to about 200 F. (93°C.); preferably from about 80 F. (27°C.) to about 140 F. (60°C.); more preferably about 110°F. (43°C.). The quench device may be operated at a pressure that is from about 2 bars (200 kPa) to about 4.5 bars (450 kPa); preferably from about 2 bars (200 kPa) to about 3.77 bars (377 kPa); and more preferably at about 2.4 bars (240 kPa).

The step of contacting the olefins stream (exiting the dehydration reactor) with a quench medium removes water from the olefins stream. In one embodiment, the step of contacting the olefins stream with a quench medium removes 95 wt. % or more of the water, preferably 98 wt. % or more, more preferably 99 wt. % or more from the olefins stream based upon the total amount of water in the olefins stream before the olefins stream is quenched.

In one embodiment, a majority of catalyst fines present in the olefins stream may be removed in the quench bottoms stream of the quench device. The term "majority" means more than 50%. According to one embodiment, the weight of the catalyst fines in the quench bottoms stream is about 5 wt. % or less, preferably about 2 wt. % or less, more preferably about 0.1 wt. % or less based on the total weight of the quench bottoms stream. According to one embodiment, the weight of catalyst fines in the quench bottoms stream is about 10 wppm or greater based upon the total weight of the quench bottoms stream.

There is another embodiment according to any process disclosed herein wherein the step of contacting the olefins stream with a quench medium removes 5 wt. % or more, preferably 50 wt. % or more, more preferably 75 wt. % or more, most preferably about 80 wt. % of the carbon dioxide from the olefins stream based upon the total amount of carbon dioxide in the olefins stream before the olefins stream is quenched.

A portion of oxygenates (e.g., aldehydes and/or ketones), in one embodiment, are present in the quench bottoms stream. According to one embodiment, the step of contacting the olefins stream with a quench medium removes from about 25 wt. % to about 95 wt. %, preferably from about 40 wt. % to about 90 wt. %, more preferably from about 50 wt. % to about 85 wt. %, of the aldehydes and/or ketones from the olefins stream based upon the total amount of aldehydes and/or ketones in the olefins stream before the olefins stream is quenched.

Oxygenates, including but not limited to unreacted ethanol feed, particularly bioethanol feed, is found in the olefins stream exiting the dehydration reactor typically when oxygenate conversion to olefin in the dehydration reactor is less than 100%. In one embodiment, the olefins stream further comprises one or more alcohols other than ethanol. Oxygenates, including but not limited to unreacted bioethanol, are at least partially removed from the olefins stream in the quench column. The step of contacting the olefins stream with a quench medium removes 90 wt. % or more, preferably 95 wt. % or more, more preferably 98 wt. % or more, most preferably 99 wt. % or more of alcohols from the olefins stream based upon the total amount of alcohol in the olefins stream before the olefins stream is quenched.

At least a portion of the quenched olefins stream which provides the ethylene fraction A used in the present invention is gaseous after at least one step of quenching, typically comprises light olefin(s) including ethylene, propylene and butylene, dimethyl ether, methane, carbon monoxide, ethane, propane, and any water and unreacted oxygenate(s) such as alcohol (including methanol) that are not condensed during the operation of the quench device. According to some embodiments of the present invention, not all carbon dioxide is removed from the reactor by the step contacting the olefins stream with a quench medium.

### Step (c): Optional Upstream Compression

If the ethylene-containing feedstream C2 or the portion thereof which is fed to the second reactor has a pressure which is compatible with the operating pressure of the second reactor and if the slipstream B 1 or the portion thereof which is fed to the first reactor has a pressure which is compatible with the operating pressure of the first reactor, then the compression step (c) on the fraction A may be omitted.

If the ethylene-containing fraction A exiting the top of the quench column has a pressure lower than what would be required for either or both of the first and second reactors, then the ethylene-containing fraction A exiting the top of the quench column may be subjected to a compression (e.g., passed through an optional upstream compressor 45 in FIG. 1-8) to form the compressed stream A1.

The resulting pressure of the compressed stream A1 may be at least 1 bars (100 kPa), or at least 1.1 bars (110 kPa), or at least 2 bars (200 kPa), or at least 3 bars (300 kPa), or at least 4 bars (400 kPa). The resulting pressure of the compressed stream A1 may be 25 bars or less (2500 kPa or less), or 20 bars or less (2000 kPa or less), or at most 15 bars or less (1500 kPa or less).

The optional upstream compression in step (c) may be performed in a single stage of compression or may be performed in several stages, either in a multi-stage gas compressor or in several compressors. The compression ratio at each compression stage is such that the temperature at the exit of the compression stage is advantageously of at most 150 °C, preferably of at most 120 °C and more preferably of at most 100 °C. The gas which exits the upstream compression stage may be advantageously cooled down afterwards by indirect cooling with a cooling media. The cooling media is advantageously chosen among cooling tower water, cold water, atmospheric air and colder gas issued from the process. The cooling media may be preferably chosen among cooling tower water and atmospheric air. The cooling fluid is more preferably cooling tower water.

The compressed stream A1 may be advantageously cooled down under 50°C, preferably under 48°C and more preferably under 45°C but advantageously not under 0°C, preferably not under 5°C and more preferably not under 10°C. At the end of the cool down, some condensates (e.g., water) may be produced. If some condensate is produced, it is preferably separated.

If the dehydration conditions are such that the ethylene-containing fraction A exiting the top of the quench column is superatmospheric (such as the ethylene-containing fraction A exiting the top of the quench column has a pressure that would be acceptable for either or both of the first and second reactors, a compression step before division into the slipstreams B1 and B2 may not be necessary and can be omitted. For example, if the dehydration conditions use a liquid-phase reaction operating at a superatmospheric pressure of from 2 bars (200 kPa) to 25 bars (2500 kPa), the fraction A is not subjected to a compression step before it is divided into B1 and B2.

### Step (d): Dividing to make two slipstreams B1 and B2

The fraction A ('as is', that is to say, not subjected to compression) or the compressed stream A1 comprising ethylene is divided into at least two portions. According to preferred embodiments of the process according to the invention, the fraction A or A1 is preferably split (divided) into at least two portions of the same composition, that is to say, into slipstreams B 1 and B2 having the same organic composition.

According to alternate less-preferred embodiments of the process according to the invention, the fraction A or the compressed stream A1 may be fractioned into at least two portions of different compositions, that is to say, into fractions B1' and B2' having different organic compositions.

The term "divided" (or "division") in the phrases "fraction A is divided into" or "stream A1 is divided into" is understood to mean, for the purpose of the invention, the splitting of fraction A or stream A1 into two or more sub-mixtures in such a way that all the sub-mixtures are characterized, at the specified pressure range, by a composition which is comprised in the range defined by the composition of the initial fraction A or stream A1 at the bubble point and by the composition of fraction A or stream A1 at the dew point.

After the split point T however, the compositions of the slipstreams B1 and B2 may be altered independently in such a way that their respective compositions (especially in types of organic components and their content) differ before their entry in their respective reactors.

### Step (f): Oxygenates Removal

In preferred embodiments, the reactions taking place in the first and second reactors may not have the same sensitivity to oxygenates which are present in the olefins stream which serves to provide two feedstreams to these reactors. When the first and second reactors comprise oxychlorination and chlorination respectively, it has been found that chlorination is prone to catalyst poisoning, deactivation, or loss of productivity when exposed to oxygenates, but on the other end, oxychlorination is insensitive to oxygenates impurities. As such, it is not necessary to purify the ethylene slipstream B1 by removing some oxygenates before being directed to the first reactor (e.g., which includes oxychlorination), while oxygenates impurities are undesirable components for the chlorination and need to be removed from the ethylene slipstream B2 before being directed to the second reactor. Because of the sensitivity of the chlorination reaction to at least some oxygenates impurities present in the olefins slipstream B2 derived from ethanol dehydration, the process according to the present invention comprises performing the oxygenates removal step (f) on at least a portion of the slipstream B2 of the ethylene product before the slipstream B2 is directed to the second reactor.

In a first variant (f1) of step (f), the oxygenates removal step precedes the drying step (e).

In a second variant (f2) of step (f), the oxygenates removal step follows the drying step (e). In such instances, step (f2) may include a fractionation, such that a dried ethylene-containing stream produced by the drying step (e) is fractionated. The fractionation of the dried ethylene-containing stream may include forming a light fraction containing most of the ethylene and a heavy fraction containing most of oxygenates.

In a third variant (f3) of step (f), the oxygenates removal step comprises carrying out the oxygenates removal step (f1) which precedes the drying step (e) and the oxygenates removal step (f2) which follows the drying step (e). In such instances, the oxygenates removal step (f2) preferably includes a fractionation, and/or the oxygenates removal step (f1) may exclude a fractionation.

In a fourth variant of step (f), the process may include a combined sulfur and oxygenates removal step (f4). Step (f4) may be carried out before the drying step (e).

Examples of oxygenates which may be present in the olefins stream originating from dehydration of ethanol may include: aldehydes (e.g, acetaldehyde), ketones, carboxylic acids and their corresponding esters, and/or ethers.

The oxygenates removal step (f) in any of its variants may include one or more of the steps selected from the group consisting of:
1/ passing the slipstream B2 or a portion thereof through a solid adsorbent (particularly a molecular sieve) so as to adsorb of at least a portion of the oxygenates in the solid adsorbent;
2/ contacting the slipstream B2 or a portion thereof with a reducing agent such as a metal hydride (particularly an alkali metal borohydride or borohydrate) so as to convert at least a portions of oxygenates (such as aldehydes and ketones) to alcohols; and/or an alkali metal bisulfite; and
3/ passing the slipstream B2 or a portion thereof through a fractionation column so as to remove of at least a portion of the oxygenates in a column bottoms.

The step for removing oxygenates from the slipstream B2 comprising ethylene and undesirable oxygenates may include contacting it with a solid adsorbent. The oxygenates removed from the slipstream B2 are preferably dimethyl ether and/or acetaldehyde, and more preferably the oxygenate is dimethyl ether. Desirably, the solid adsorbent is a molecular sieve or metal oxide. Preferably, the solid adsorbent is a molecular sieve. The small, medium and large pore molecular sieves have from a 4-ring to a 12-ring or greater framework-type. The molecular sieve preferably has a framework structure of at least 8 rings. In a more preferred embodiment, the molecular sieves have 8-, 10-or 12-ring structures or larger and an average pore size in the range of from about 3 A to 15 A. The molecular sieves can be amorphous, crystalline, or a combination thereof. Examples of molecular sieves include zeolite as well as non-zeolite molecular sieves, which are of the large, medium or small pore type. Non-limiting examples of these molecular sieves include the small pore molecular sieves, AEI, AFT, APC, ATN, ATT, ATV, AWW, BIK, CAS, CHA, CHI, DAC, DDR, ED1, ER1, GOO, KFI, LEV, LOV, LTA, MON, PAU, PHI, RHO, ROG, THO, and substituted forms thereof; the medium pore molecular sieves, AFO, AEL, EUO, HEU, FER, MEL, MFI, MTW, MTT, TON, and substituted forms thereof; and the large pore molecular sieves, EMT, FAU, and substituted forms thereof. Other molecular sieves include ANA, BEA, CFI, CLO, DON, GIS, LTL, MER, MOR, MWW and SOD. Preferred types of molecular sieves include faujasites, pentasils, mordenite, beta, VPI, MCM, and substituted aluminophosphates such as SAPO, MeAPO, ELAPO, and ELAPSO. Also preferably, the molecular sieve is a zeolite. Non-limiting examples of the preferred molecular sieves include zeolite X, zeolite Y, VPI-5, MCM-41, ZSM-5, ZSM-11, ZSM-14, ZSM-17, ZSM-18, ZSM-20, ZSM-31, ZSM-34, ZSM-41 and ZSM-46. In one preferred embodiment, the molecular sieve used in this step is zeolite X or zeolite Y. Of these, zeolite X or Y is preferred, with zeolite X being particularly preferred. The solid adsorbent can be kept in continuous use by regenerating following contacting with the provided olefin stream.

The contact with the slipstream B2 or portion thereof with a hydride and more particularly a borohydride such as sodium or lithium borohydride, typically results in removing at least some aldehydes such as acetaldehyde present in this ethylene-containing stream by a reduction reaction which can be carried out in an alkaline medium. In particular, sodium borohydride (NaBH₄) is generally used in basic aqueous solution such as a sodium hydroxide solution whose concentration may vary between 0.1 M/l and 10 M/l and preferably between 1 and 5 M/l. The concentration of NaBH₄ in this alkaline solution is generally between 0.01 M/l and the solubility limit of NaBH₄ in this alkaline solution; however, the concentration of NaBH₄ is preferably between 0.1 and 1 M/l. For this reduction step, the temperature conditions may be between 10 °C and 70 °C, preferably between 15 °C and 35 °C. With regard to the pressure conditions, these are not critical and are usually between 1 and 10 bars (between 100 and 1000 kPa) in such treatment, but may be higher. This treatment usually performed at a pressure which is lower or about equal that of the pressure conditions for the second reactor. When the oxygenate removal step comprises a contact with a metal hydride, it is preferably followed by a drying step (e).

The step for removing oxygenates may include contacting the slipstream B2 comprising ethylene and undesirable oxygenates with an alkali metal bisulfite, more particularly sodium bisulfite. The contact typically results in removing at least some aldehydes such as acetaldehyde present in this ethylene-containing stream. When the treatment is carried out with sodium bisulfite, the operation is advantageously carried out at a pH which is controlled and, in all cases, lying between the values of 5 and 9 in order to avoid the formation of excessively large quantities of SO₂ in the reaction mixture. The pH of the mixture will be preferably maintained between the values included between 6.5 and 8.5, more preferably between 7 and 8, most preferably at about 7.5. The pH control may be ensured by any means allowing this effect to be achieved. A means which has given good results consists in adding sodium sulfite in sufficient quantities to attain the desired pH.

When the oxygenates removal step (f) is carried out with an aqueous solution of sodium bisulfite, the NaHSO₃ concentration is generally between 0.01 M/l and the solubility limit of this compound in water in the operating conditions of temperature and pressure However, the concentration of sodium bisulfite is preferably between 0.05 and 1 M/l. The bisulfite treatment may be carried out using an aqueous solution containing only the bisulfite or may be carried out in the presence of other components which do not affect the proper progress of the reaction. Thus, an embodiment may comprise combining the operation of drying the slipstream B2 with the alkali metal bisulfite treatment. The alkali metal bisulfite treatment can be carried out in temperature conditions which are usually between -15 °C and 40 °C, preferably between -10 °C and 35 °C. With regard to the pressure conditions, these are not critical and are usually between 1 and 10 bars (between 100 and 1000 kPa) in such treatment, although higher pressures may be used. This treatment usually performed at a pressure which is lower or about equal that of the pressure conditions for the second reactor.

For the oxygenates removal step (f), it is preferred to use contacting the slipstream B2 or portion thereof with a hydride, compared to contacting the slipstream B2 or portion thereof with an alkali metal bisulfite or with a molecular sieve.

For the oxygenates removal step (f), a fractionation may be used, in which case most of the oxygenates which are heavier than ethylene get separated and recovered in the column bottoms. The fractionation used for oxygenates removal may also be effective to remove non-oxygenated hydrocarbons with 3 or more carbon atoms (C₃⁺ hydrocarbons), such as propylene, propane; the oxygenates and non-oxygenated C₃⁺ hydrocarbons which are removed from the ethylene stream are found in the fractionation bottoms. The fractionation design and operating conditions are selected in a manner such that the overhead stream comprising the majority of ethylene which serves as feedstock to the chlorination reactor has a propylene content of 2 ppm or less. A preferred propylene content in the overheads stream from such fractionation is 1 ppm or less.

### Step (e): Drying

The process further comprises carrying out a drying step (e) on at least a portion of the slipstream B2. The drying step (e) may include passing the slipstream B2 through a drying unit to obtain a dried ethylene-containing stream.

The drying step (e) may include passing an oxygenates-lean stream resulting from the oxygenates removal step (f1) through a drying unit in order to obtain at least a dried ethylene-containing stream.

The drying step (e) may include passing a desulfurized oxygenates-lean stream resulting from the combined sulfur and oxygenates removal step (f4) through a drying unit in order to obtain at least a dried ethylene-containing stream.

Any suitable means may be used for drying. The drying step (e) may include compression, fractionation, solid or liquid adsorption, or combinations of two or more of these techniques. The drying step (e) should be effective in knocking some water out from at least a portion of the slipstream B2. A solid adsorption for example may include a molecular sieve adsorption.

When the drying step (e) includes a compression, such compression, also identified as 'downstream compression' since it is carried out after the split point T, may be performed in a single stage of compression or may be performed in several stages, either in a multi-stage gas compressor or in several compressors. The compression ratio at each compression stage is such that the temperature at the exit of the compression stage is advantageously of at most 150°C, preferably of at most 120°C and more preferably of at most 100°C. The gas which exits the compression stage is afterwards advantageously cooled down by indirect cooling with a cooling medium. The cooling medium is advantageously selected from the group consisting of cooling tower water, cold water, atmospheric air and colder gas issued from the process. The cooling medium is preferably selected from the group consisting of cooling tower water and atmospheric air. The cooling medium is more preferably cooling tower water.

The gas is advantageously cooled down under 50°C, preferably under 48°C and more preferably under 45°C but advantageously not under 0°C, preferably not under 5°C and more preferably not under 10°C. At the end of the cool down, some condensates (e.g., water) could be produced. If some condensates are produced, they are preferably separated.

When the drying step (e) includes a fractionation, such fractionation may be performed by a distillation column in which the heavies (e.g., water) are collected at the bottoms and ethylene is mainly separated into a light fraction.

When the drying step (e) includes an adsorption, such adsorption may be performed by solid or liquid adsorption to remove water from the ethylene-containing stream. In the solid drying system, the olefins stream is contacted with a solid adsorbent to further remove water to very low levels. Typically, the adsorption step is carried out in one or more fixed beds containing a suitable solid adsorbent.

Adsorption is useful for removing water and also oxygenates to very low concentrations, and for removing oxygenates that may not normally be removed by using other treatment systems. Preferably, an adsorbent system may have multiple adsorbent beds. Multiple beds allow for continuous separation without the need for shutting down the process to regenerate the solid adsorbent. By way of example and not by limitation, a three bed system typically has one bed that is on-line, one bed regenerated off-line, and a third bed on stand-by.

The specific adsorbent solid or solids used in the adsorbent beds depends on the types of contaminants being removed. Non-limiting examples of solid adsorbents for removing water and various polar organic compounds, such as oxygenates and absorbent liquids, include calcium chloride, aluminas, silica, 3A molecular sieves, 4A molecular sieves, and alumino-silicates. Beds containing mixtures of these sieves or multiple beds having different adsorbent solids are used to remove water, as well as a variety of oxygenates in one embodiment.

In an embodiment of the present invention, one or more adsorption beds are arranged in series or parallel. In one example of a series arrangement, a first bed is used to remove the smallest and most polar molecules, which are the easiest to remove. Subsequent beds for removing larger less polar oxygenated species are next in series. As a specific example of one type of arrangement, water is first selectively removed using a 3A molecular sieve. This bed is then followed by one or more beds containing one or more less selective adsorbents such as a larger pore molecular sieve, e.g., 13x ; and/or a high surface area active alumina.

In another embodiment, the first bed is a 3.6 A molecular sieve capable of selectively separating both water and methanol from the hydrocarbons in the olefin stream. This first bed can then be followed by one or more 13x; or active alumina beds as described above.

The adsorbent beds can be operated at ambient temperature or at elevated temperature as required, and with either upward or downward flow. Regeneration of the adsorbent materials can be carried out by conventional methods including treatment with a stream of a dry inert gas such as nitrogen at elevated temperature.

In the liquid adsorption drying system, a water absorbent is used to remove water from the ethylene-containing slipstream B2 or portion thereof. The water absorbent can be any liquid effective in separating water from an olefin stream.

When the drying step (e) includes a downstream compression and an adsorption, the compression is preferably carried out prior to the adsorption.

When the drying step (e) includes an adsorption and a fractionation, the adsorption is preferably carried out prior to the fractionation.

When the drying step (e) includes a downstream compression, an adsorption, and a fractionation, the downstream compression is preferably carried out first in the series, the adsorption is carried out second and the fractionation is carried out third.

Regardless of which technique is used in the drying step (e), the drying step (e) produces a dried ethylene-containing stream.

Preferably, the dried ethylene-containing stream contains less than about 100 wppm water, more preferably less than about 10 wppm, and most preferably less than 1 wppm based upon the weight of the olefin product stream.

Carrying out the drying step (e) and oxygenates removal step (f) are generally effective in generating an ethylene-containing feedstream C2 suitable as a feedstock to a chlorination reactor. The feedstream C2 is preferably leaner in water and in oxygenates than the slipstream B2, that is to say, the feedstream C2 has a lower content in water and in oxygenates than the slipstream B2 which was formed at the point T of division.

Preferably less than about 10 wppm, more preferably less than about 5 wppm, and most preferably less than about 1 wppm dimethyl ether is present in the dried ethylene-containing stream.

### Step (g): Conversion of Ethylene in First Reactor

The process comprises step (g): conveying the first slipstream B 1 or a portion thereof to a first reactor to convert at least a portion of the ethylene to at least one (first) ethylene derivative and to generate a first product stream P1 comprising said formed (first) ethylene derivative.

The process may comprise further conveying at least a portion of the slipstream B2, which may have been subjected to an optional downstream compression, to drying, to optional sulfur removal, to oxygenates removal, or to any combinations of two or more thereof, to the first reactor where the ethylene contained herein may be converted at least in part to the first ethylene derivative. For example, a portion of slipstream B2 which has been subjected to an optional sulfur removal and to an oxygenates removal may be directed to the first reactor instead of being directed to the second reactor. Another example may be when a portion of the feedstream C2 (a portion of slipstream B2 which has been subjected to oxygenates removal and to drying) is directed to the first reactor instead of being directed to the second reactor.

The process may further comprise conveying at least a portion P2' of the product stream P2 (exiting the second reactor) which comprises unconverted ethylene to the first reactor where some of the remaining ethylene will be converted to the first ethylene derivative. The portion P2' of the product stream P2 may contain the same (first) ethylene derivative which is formed in the first reactor and present in product stream P1 or may contain another (second) ethylene derivative different than the (first) ethylene derivative formed in the first reactor. This arrangement of reactors is an example of an operation in series. The reactor which is first in the series is preferably the one which comprises reaction conditions more susceptible to be negatively impacted by undesirable components (e.g., sulfur-containing compounds, oxygenates, and/or water). The ethylene derivative formed in the second reactor may be removed at least in part from the portion P2' of the product stream P2 which is fed to the first reactor. Alternatively, the portion P2' of the product stream P2 is not subjected to a separation to remove the (second) ethylene derivative formed in the second reactor before P2' is fed to the first reactor.

The (first) ethylene derivative formed in the first reactor is preferably DCE. The first reactor preferably comprises an oxychlorination reaction.

### Oxychlorination reaction in step (g)

The oxychlorination reaction is advantageously performed in the presence of a catalyst comprising active elements including copper deposited on an inert support. The inert support is advantageously chosen from alumina, silica gels, mixed oxides, clays and other supports of natural origin. Alumina constitutes a preferred inert support.

Catalysts comprising active elements which are advantageously at least two in number, one of which is copper, are preferred. Among the active elements other than copper, mention may be made of alkali metals, alkaline-earth metals, rare-earth metals and metals from the group consisting of ruthenium, rhodium, palladium, osmium, iridium, platinum and gold. The catalysts containing the following active elements are particularly advantageous: copper/magnesium/potassium, copper/magnesium/sodium; copper/magnesium/ lithium, copper/magnesium/caesium, copper/magnesium/sodium/lithium, copper/ magnesium/potassium/lithium and copper/magnesium/caesium/lithium, copper/ magnesium/sodium/potassium, copper/magnesium/sodium/caesium and copper/ magnesium/potassium/caesium. The catalysts described in Patent Applications EP-A 255 156, EP-A 494 474, EP-A-657 212, and EP-A 657 213, incorporated by reference, are most particularly preferred.

The copper content, calculated in metal form, is advantageously between 30 and 90 g/kg, preferably between 40 and 80 g/kg and particularly preferably between 50 and 70 g/kg of catalyst.

The magnesium content, calculated in metal form, is advantageously between 10 and 30 g/kg, preferably between 12 and 25 g/kg and particularly preferably between 15 and 20 g/kg of catalyst.

The alkali metal content, calculated in metal form, is advantageously between 0.1 and 30 g/kg, preferably between 0.5 and 20 g/kg and particularly preferably between 1 and 15 g/kg of catalyst.

The Cu:Mg:alkali metal(s) atomic ratios are advantageously 1:0.1-2:0.05-2, preferably 1:0.2-1.5:0.1-1.5 and particularly preferably 1:0.5-1:0.15-1.

Catalysts having a specific surface area, measured according to the BET method with nitrogen that is advantageously between 25 m²/g and 300 m²/g, preferably between 50 m²/g and 200 m²/g and particularly preferably between 75 m²/g and 175 m²/g, are particularly advantageous.

The catalyst may be used in a fixed bed or in a fluidized bed. This second option is preferred. The oxychlorination process is operated under the range of the conditions usually recommended for this reaction. The temperature is advantageously between 150°C and 300°C, preferably between 200°C and 275°C and most preferably from 215°C to 255°C. The pressure is advantageously above atmospheric pressure. Values of between 2 and 10 bars (between 200 and 1000 kPa) absolute gave good results. The range between 4 and 7 bars absolute (between 400 and 700 kPa) is preferred. This pressure may be usefully adjusted in order to attain an optimum residence time in the reactor and to maintain a constant rate of passage for various operating speeds. The usual residence times range from 1 to 60 seconds, preferably from 10 to 40 seconds.

The source of oxygen for this oxychlorination may be air, pure oxygen or a mixture thereof, preferably pure oxygen. The latter solution, which allows easy recycling of the unconverted reactants, is preferred.

The reactants may be introduced into the bed by any known device. It is generally advantageous to introduce the oxygen separately from the other reactants for safety reasons. These safety reasons also require the gaseous mixture leaving the reactor or recycled thereto to be kept outside the limits of inflammability at the pressures and temperatures in question. It is preferable to maintain a so-called rich mixture, that is to say containing too little oxygen relative to the fuel to ignite. In this regard, the abundant presence (> 2 vol %, preferably > 5 vol %) of hydrogen would constitute a disadvantage given the wide range of inflammability of this compound.

The hydrogen chloride/oxygen ratio used is advantageously between 3 and 6 mol/mol. The ethylene/hydrogen chloride molar ratio is advantageously between 0.4 and 0.6 mol/mol.

The chlorinated product stream P1 obtained contains mainly DCE and may also contain small amounts of by-products such as 1,1,2-trichloroethane.

### Step (h): Conversion of Ethylene in Second Reactor

The process comprises step (h): conveying the ethylene-containing feedstream C2 or a portion thereof to a second reactor to convert ethylene to at least one ethylene derivative and to generate a second product stream P2 comprising said formed ethylene derivative.

The formed ethylene derivative in product stream P2 may be different than the ethylene derivative which is formed in step (g) or preferably may be the same ethylene derivative formed in step (g).

The ethylene derivative formed in the second reactor is preferably DCE. The second reactor preferably comprises a chlorination reaction.

### Chlorination reaction in step (h)

The chlorination reaction (usually called direct chlorination) in the second reactor is advantageously carried out in a liquid phase (preferably comprising mainly DCE) containing a dissolved catalyst such as FeCl₃ or another Lewis acid. It is possible to advantageously combine this catalyst with cocatalysts such as alkali metal chlorides. A pair which has given good results is the complex of FeCl₃ with LiCl (lithium tetrachloroferrate - as described in Patent Application NL 6901398).

The amounts of FeCl₃ advantageously used are around 1 to 30 g of FeCl₃ per kg of liquid stock. The molar ratio of FeCl₃ to LiCl is advantageously of the order of 0.5 to 2.

In addition, the chlorination reaction may be performed in a chlorinated organic liquid medium. More preferably, this chlorinated organic liquid medium, also called liquid stock, comprises mainly of DCE.

The chlorination reaction according to the invention is advantageously performed at temperatures between 30 and 150°C. Good results are obtained regardless of the pressure both at a temperature below the boiling point (chlorination process under subcooled conditions) and at the boiling point itself (process for chlorination at boiling point).

When the chlorination reaction scheme is a chlorination process under subcooled conditions, it gave good results by operating at a temperature which was advantageously greater than or equal to 50°C and preferably greater than or equal to 60°C, but advantageously less than or equal to 80°C and preferably less than or equal to 70°C, and with a pressure in the gaseous phase advantageously greater than or equal to 1 and preferably greater than or equal to 1.1 bars absolute (110 kPa), but advantageously less than or equal to 20 bars (2000 kPa), preferably less than or equal to 10 bars (1000 kPa), and particularly preferably less than or equal to 6 bars absolute (600 kPa).

A process for chlorination at boiling point may be preferred to usefully recover the heat of reaction. In this case, the reaction advantageously takes place at a temperature greater than or equal to 60°C, preferably greater than or equal to 70°C and particularly preferably greater than or equal to 85°C, but advantageously less than or equal to 150°C and preferably less than or equal to 135°C, and with a pressure in the gaseous phase advantageously greater than or equal to 0.2, preferably greater than or equal to 0.5, particularly preferably greater than or equal to 1.1 bars absolute (110 kPa) and more particularly preferably greater than or equal to 1.3 bars absolute (130 kPa), but advantageously less than or equal to 10 bars absolute (1000 kPa) and preferably less than or equal to 6 bars absolute (600 kPa).

The chlorination process may also be a hybrid loop-cooled process for chlorination at boiling point. The expression "hybrid loop-cooled process for chlorination at boiling point" is understood to mean a process in which cooling of the reaction medium is carried out, for example, by means of an exchanger immersed in the reaction medium or by a loop circulating in an exchanger, while producing in the gaseous phase at least the amount of DCE formed. Advantageously, the reaction temperature and pressure are adjusted for the DCE produced to leave in the gaseous phase and for the remainder of the heat from the reaction medium to be removed by means of the exchange surface area.

The feedstream C2 or a portion thereof submitted to the chlorination and also the molecular chlorine (itself pure or diluted) may be introduced, together or separately, into the reaction medium by any known device. A separate introduction of the feedstream C2 or portion thereof submitted to the chlorination may be advantageous in order to increase its partial pressure and facilitate its dissolution which often constitutes a limiting step of the process.

The molecular chlorine is added in a sufficient amount to convert most of the ethylene and without requiring the addition of an excess of unconverted chlorine. The chlorine/ethylene ratio used is preferably between 1.2 and 0.8 and particularly preferably between 1.05 and 0.95 mol/mol.

The chlorinated products obtained contain mainly DCE and also small amounts of by-products such as 1,1,2-trichloroethane or small amounts of ethane or methane chlorination products.

The separation of the DCE product obtained from the stream of products derived from the chlorination reactor may be carried out according to known techniques and in general makes it possible to exploit the heat of the chlorination reaction. It is then preferably carried out by condensation and gas/liquid separation.

The unconverted products (methane, ethane, carbon monoxide, nitrogen, oxygen and hydrogen) are then advantageously subjected to an easier separation than what would have been necessary to separate pure ethylene starting from the initial mixture.

### Ethylene Derivative Compound

In some embodiments, the at least one ethylene derivative compounds generated in step (g) and (h) are different.

In other embodiments, the at least one ethylene derivative compounds generated in step (h) and (g) are the same.

In a particular embodiment, the at least one ethylene derivative compound generated in both steps (h) and (g) includes or is 1,2-dichloroethane (DCE).

### Step (i) : Optional Downstream Compression

If the ethylene-containing feedstream C2 or a portion thereof which is fed to the second reactor has a pressure lower what is acceptable for the operating pressure of the second reactor, but the slipstream B 1 or the portion thereof which is fed to the first reactor has a pressure which is compatible with the operating pressure of the first reactor, then the main compression step (c) on the fraction A may be omitted in the process; but in such instance, the process may comprise instead a secondary (downstream) compression step carried out after the formation of the second slipstream B2 and before the ethylene-containing feedstream C2 or the portion thereof is fed to the second reactor.

If the ethylene-containing feedstream C2 or a portion thereof which is fed to the second reactor has a pressure lower what is acceptable for the operating pressure of the second reactor, and the slipstream B1 or the portion thereof which is fed to the first reactor also has a pressure lower what is acceptable for the operating pressure of the first reactor, then the main compression step (c) on the fraction A may be carried out to achieve at a minimum a pressure suitable for operating in the first reactor; in such instance, the process may further comprise a secondary compression step carried out after the formation of the second slipstream B2 and before the ethylene-containing feedstream C2 or the portion thereof is fed to the second reactor.

In some embodiments, the downstream compression stage (i) is carried out after the split point T on at least a portion of the slipstream B2 before its entry into the second reactor (e.g., a chlorination reactor).

The downstream compression step (i) may comprise:
(i1) carrying out the downstream compression on the slipstream (B2) before the oxygenates removal step (f1); and/or
(i2) carrying out the downstream compression on the slipstream (B2) before the oxygenates removal step (f2) or (f3) and before drying step (e). When the process according to the present invention comprises subjecting at least a portion of the slipstream B2 or the resulting dried stream after drying step (e) to fractionation which can serve as an effective oxygenates removal step (f), the process preferably comprises performing the downstream compression (i) before such fractionation.

It should be understood that if the pressure of the slipstream B1 is too low to be suitable for the first reactor pressure conditions, it may be necessary for the process to further comprises a downstream compression step (i') being carried out after the split point T on at least a portion of the slipstream B 1 before its entry into the first reactor (e.g., an oxychlorination reactor).

The pressure of the resulting compressed stream after the downstream compression step (i) or (i') may be at least 1.1 bars (110 kPa), or at least 2 bars (200 kPa), or at least 4 bars (400 kPa). The resulting pressure of the resulting compressed stream may be at most 20 bars (2000 kPa), or at most 10 bars (1000 kPa), or at most 6 bars (600 kPa).

### Step (j') or (j): Optional Upstream and/or Downstream Desulfurization

In some embodiments, the process does not include a desulfurization step. For example, when the bioethanol is a fuel-grade ethanol in which water has been removed, generally such fuel-grade bioethanol is substantially free of sulfur (e.g., less than 1 ppm sulfur).

In other instances when the bioethanol is in hydrous form, the process of the present invention may further comprise performing a desulfurization step before either or both of the reaction steps (g) and (h) which convert ethylene to the ethylene derivative(s).

An upstream desulfurization step (j') may be carried out upstream of the split point T, for example on at least a portion of the fraction A or at least a portion of the compressed stream A1 before the creation of slipstreams B1 and B2. For example, the upstream desulfurization step (j') may comprise subjecting the fraction A or compressed stream A1 to desulfurization before forming the slipstreams B1 and B2. The technique for the upstream desulfurization step (j') may be selected from the group consisting of an alkali wash, an activated carbon adsorption, an alkali metal hydride adsorption, and combinations of two or more thereof.

A downstream desulfurization step (j) may be carried out downstream of the split point T, for example on at least a portion of the slipstream B1 and/or of the slipstream B2, and/or of the feedstream C2.

The downstream desulfurization step (j) may comprise separately subjecting at least a portion of the slipstream B1, or at least a portion of the slipstream B2, or at least a portion of the feedstream C2 (which is leaner in oxygenates and/or water than slipstream B2) to desulfurization. The technique for the downstream desulfurization step (j) may be selected from the group consisting of an alkali wash, an alkali metal hydride or hydrate, an activated carbon adsorption, and combinations of two or more thereof.

The downstream desulfurization step (j) may comprise at least one of the following variant steps:
(j1) subjecting the slipstream B2 or a portion thereof to a desulfurization technique selected from the group consisting of an alkali wash, an activated carbon adsorption, an alkali metal hydride adsorption, and combinations of two or more thereof, said step (j1) being carried out before the drying step (e) and before the oxygenates removal step (f);
(j2) subjecting the slipstream B2 or a portion thereof to a desulfurization technique selected from the group consisting of an alkali wash, an activated carbon adsorption, an alkali metal hydride adsorption, and combinations of two or more thereof, said step (j2) being carried out in between the drying step (e) and the oxygenates removal step (f);
(j3) subjecting the slipstream B2 or a portion thereof during the oxygenates removal step (f1) to a desulfurization technique selected from the group consisting of an alkali wash, an alkali metal hydride adsorption, and combinations thereof;
(j4) subjecting the ethylene-containing feedstream C2 resulting from step (e) and step (f) to a desulfurization technique which includes an activated carbon adsorption;
(j5) subjecting the slipstream B1 or a portion thereof to a desulfurization technique selected from the group consisting of an alkali wash, an activated carbon adsorption, an alkali metal hydride adsorption, and combinations of two or more thereof; or
combinations of two or more thereof.

If the upstream desulfurization step (j') is carried out on the ethylene-containing fraction A or compressed stream A1 before the split point T, then a downstream desulfurization step (j) is preferably not carried out on any of the slipstreams B1 and B2 and/or on feedstream C2. However supplemental downstream desulfurization may be performed on either or both of slipstreams B1 and B2 or any portion thereof if necessary. If an upstream desulfurization step (j') is not carried out on the ethylene-containing fraction A or compressed stream A1, then at least one of the slipstreams B1 and B2 is subjected to a downstream desulfurization step (j) to remove at least a portion of the sulfur-containing compounds. If both slipstreams B 1 and B2 are subjected to a downstream desulfurization step (j), they are separately desulfurized in different downstream desulfurization units.

In some variants of the desulfurization step (j), the downstream desulfurization step (j3) combines desulfurization with the oxygenates removal step (f), preferably with the oxygenates removal step (f1) which is carried out before drying step (e). In this embodiment, the sulfur removal step and the oxygenates removal step are carried out in the same unit (see for example unit 100 in FIG. 3 and FIG. 6).

The desulfurization technique may be selected from the group consisting of an alkali wash, an alkali metal hydride adsorption, a reaction with a peroxide (such as H₂O₂), an activated carbon adsorption, a selective absorption, a selective adsorption, and combinations of two or more thereof. The desulfurization technique for removal of sulfur compounds preferably excludes hydrogenation.

An alkali wash may include scrubbing with an aqueous solution of an alkali metal hydroxide (e.g., NaOH) or with an aqueous solution of alkaloamine (e.g., monoethanolamine or diethanolamine).

An alkali metal hydride adsorption may include similar conditions of operation as those described for the oxygenates removal step (f).

A reaction with a peroxide (especially with H₂O₂) may include formation of oxidized sulfur compounds such as sulfones and/or sulfoxides which are heavy compounds.

Although less preferred, a selective absorption or adsorption in a desulfurization step may include contacting the ethylene stream with a liquid selective absorbent or solid selective adsorbent. Example of solid selective adsorbent includes zinc oxide optionally doped with a metal or an activated alumina with high Lewis acidity (e.g., SelexSorb™ by Alcoa). Example of liquid selective absorbent includes dimethyletherpolyethyleneglycol.

An activated carbon adsorption may include contacting the ethylene stream with an activated carbon having a specific surface area of at least 100 m²/g, or at least 150 m²/g, preferably between 200 and 2500 m²/g. Step (k): Conversion of Ethylene Derivative in Third Reactor

In some additional embodiments, the process may further comprise:
(k) passing at least the first product stream P1, the second product stream P2, portions thereof, or any combination thereof through a third reactor under suitable conditions to generate at least one first compound derived therefrom. The at least one first compound derived therefrom is preferably different from the ethylene derivative(s) formed in steps (g) and (h).

In some embodiments, the ethylene derivative compounds formed in steps (h) and (g) are the same. The process may further comprise: mixing at least a portion of the product stream P1 comprising the ethylene derivative compound formed in step (g) and at least a portion of the product stream P2 comprising the same ethylene derivative compound formed in step (h), and passing such mixture in the third reactor for further conversion to a first compound derived therefrom which is different than the ethylene derivative compound formed in steps (h) and (g). The ethylene derivative compounds formed in steps (h) and (g) may be independently isolated from the product streams P1 and P2 or may be isolated from a mixture of product streams P 1 and P2, before being passed to the third reactor. The process may further comprise: mixing the separately isolated ethylene derivative compounds, and passing such mixture in the third reactor.

In some embodiments, the ethylene derivative compounds formed in steps (h) and (g) are different. The process may further comprise: mixing at least a portion of the product stream P1 comprising a first ethylene derivative compound formed in step (g) and at least a portion of the product stream P2 comprising a second (different) ethylene derivative compound formed in step (h), and passing such mixture in the third reactor.

Or the process may further comprise: isolating the first ethylene derivative compound formed in step (g) from product stream P1 to form an isolated stream of the first ethylene derivative compound; and separately isolating the second ethylene derivative compound formed in step (g) from product stream P2 to form an isolated stream of the second ethylene derivative compound; optionally, mixing at least a portion of the isolated stream of the first ethylene derivative compound and at least a portion of the isolated stream of the second ethylene derivative compound; and passing such mixture or separately passing the isolated streams of the first and second ethylene derivative compounds in the third reactor.

Or the process may omit to separately the isolation step(s) of the ethylene derivative compound(s) from product streams P1 and P2; and comprises mixing at least a portion of the product stream P1 comprising the first ethylene derivative compound and at least a portion of the product stream P2 comprising the second (different) ethylene derivative compound, and passing such mixture to the third reactor.

Or the process may further comprise: passing the first ethylene derivative compound formed in step (g) (either via product stream P 1 or via an isolated stream from product stream P1) to a third reactor under suitable conditions to generate at least one compound derived therefrom; and separately passing the second (different) ethylene derivative compound formed in step (h) (either via product stream P2 or via an isolated stream from product stream P2) to another third reactor under suitable conditions to generate another compound derived therefrom.

In preferred embodiments, when the same ethylene derivative compound formed in steps (h) and (g) is 1,2-dichloroethane, the third reactor in such instance preferably comprise 1,2-dichloroethane cracking conditions, and the at least one first compound derived therefrom exiting the third reactor is vinyl chloride. The process may further comprise: mixing at least a portion of the product stream P1 comprising 1,2-dichloroethane and at least a portion of the product stream P2 comprising 1,2-dichloroethane, and subjecting such mixture to 1,2-dichloroethane cracking conditions to produce vinyl chloride.

In some embodiments, the process may further comprise combining at least a portion of the 1,2-dichloroethane formed in step (h) and at least a portion of the 1,2-dichloroethane formed in step (g), and passing such combination to the third reactor. In some embodiments, the 1,2-dichloroethane may be isolated from the product streams P1 and P2 exiting the first and second reactors. The isolation may be carried either separately on each individual stream P1 and P2, or preferably on a mixture of product streams P1 and P2. This optional isolation step is performed before the 1,2-dichloroethane is passed through the third reactor to produce vinyl chloride.

The 1,2-dichloroethane cracking conditions in the third reactor which is fed 1,2-dichloroethane preferably include pyrolysis.

### Pyrolysis

The conditions under which the DCE cracking step may be carried out are known to persons skilled in the art. The DCE cracking can be performed in the presence or in the absence of third compounds among which can be cited the catalysts; the DCE cracking is in this case a catalytic DCE cracking. The DCE cracking is however preferably performed in the absence of third compounds and under the action of heat only; the DCE cracking is in this case often called pyrolysis.

This pyrolysis is advantageously obtained by a reaction in the gaseous phase in a tubular oven. The usual pyrolysis temperatures are between 400 and 600°C with a preference for the range between 480°C and 540°C. The residence time is advantageously between 1 and 60 seconds with a preference for the range from 5 to 25 seconds. The rate of conversion of the DCE is advantageously limited to 45 to 75 % in order to limit the formation of by-products and the fouling of the tubes of the oven.

The separation of the VC and hydrogen chloride obtained from the stream of products derived from the pyrolysis is carried out according to known modes, using any known device, in order to collect the purified VC and the hydrogen chloride. Following purification, the unconverted DCE is advantageously conveyed to the pyrolysis oven.

### Step (1): Polymerization of Vinyl Chloride

According to some embodiments, the process may further comprise a polymerization step (1). Vinyl chloride produced from step (k) may be polymerized to produce polyvinyl chloride.

Step (1) comprises polymerizing the vinyl chloride (VC) produced in step (k) to produce polyvinyl chloride (PVC) as a second compound derived therefrom.

The manufacture of PVC may be a mass, solution or aqueous dispersion polymerization process, free radical polymerization, and/or aqueous microsuspension polymerization. Any polymerization method can be used in step (1) of the present invention. Preferably, the manufacture of PVC in step (1) includes an aqueous dispersion polymerization process.

The expression 'aqueous dispersion polymerization' is understood to mean free radical polymerization in aqueous suspension as well as free radical polymerization in aqueous emulsion and polymerization in aqueous microsuspension.

The expression 'free radical polymerization' in aqueous suspension is understood to mean any free radical polymerization process performed in aqueous medium in the presence of dispersing agents and oil-soluble free radical initiators.

The expression 'aqueous microsuspension polymerization', also called 'polymerization in homogenized aqueous dispersion', is understood to mean any free radical polymerization process in which oil-soluble initiators are used and an emulsion of droplets of monomers is prepared by virtue of a powerful mechanical stirring and the presence of emulsifying agents.

The invention will now be described with reference to the drawings.

FIG. 1-8 represent various embodiments of process schemes according to the present invention. These said embodiments comprise preferred process steps (b) through (h) as well as optional steps (i) and (j) according to the present invention. The letter/number references in FIG. 1-8 correspond to those used in the present description and appending claims.

It is to be understood that any equipment or step or stream disclosed in the context of one embodiment of the present process may be used or interchanged in the context of another embodiment, unless it is explicitly stated otherwise.

In the embodiments illustrated in FIG. 1-8, each system for carrying out the process includes an oxygenates removal unit (e.g., units 60, 100, or frationation column 110), a drying unit (e.g., unit 70 or compression units 55, 55'), a first reactor 80, and a second reactor 90.

In FIG. 1-8, the first reactor 80 comprises or preferably is an oxychlorination reactor, and the second reactor 90 comprises or preferably is a chlorination reactor. As shown in FIG. 1-7, the first and second reactors may be operated in parallel, or as shown in FIG. 8, the first and second reactors may be operated in series. Some embodiments may include an optional upstream desulfurization unit (e.g., unit 50). Alternate or additional embodiments may include one or more optional downstream desulfurization units (e.g., units 95, 120, 120', 120"...). Alternate or additional embodiments of the system may include an optional upstream compressor 45 (which may be also termed 'main' or 'primary' compressor") and/or one or more optional downstream compression units, such as units 55, 55', 55"... (which may be also termed 'supplemental' or 'secondary' compressors).

In the process carried out in any of the systems of FIG. 1-8, an ethylene-containing fraction A which is produced via dehydration of bioethanol and which exits a quench device (not illustrated) is divided into two slipstreams B1 and B2 (via split point 'T'). Slipstreams B1 and B2 are thereafter subjected to different purification schemes before they are fed to their respective reactor: oxychlorination and chlorination. The ethylene-containing fraction A may be compressed in the optional upstream compressor 45 to form the compressed stream A1 before it is divided into slipstreams B1 and B2.

FIG. 1 illustrates a first system for carrying out the process according to the present invention. This first system includes the oxygenates removal unit 60, the drying unit 70, the first (oxychlorination) reactor 80, and the second (chlorination) reactor 90. In some variants, the first system illustrated in FIG. 1 may further include an optional upstream desulfurization unit 50. In alternate or additional variants, the system illustrated in FIG. 1 may further include the optional upstream compressor 45 and an optional downstream compressor 55.

The process for making at least one ethylene derivative carried out in the first system illustrated by FIG. 1 comprises the following steps:
Step (f1): removing at least a portion of the oxygenates from slipstream B2 in oxygenates removal unit 60 before drying step (e) in drying unit 70; and
Step (i1): carrying out a secondary compression on the slipstream (B2) before the oxygenates step (f) in oxygenates removal unit 60.

The process may optionally comprise at least one desulfurization:
Step (j'): subjecting the fraction A or the compressed stream A1 exiting unit 45 to desulfurization in desulfurization unit 50 before forming the slipstreams B1 and B2, wherein the desulfurization unit 50 includes a desulfurization technique selected from the group consisting of an alkali wash, an activated carbon adsorption, an alkali metal hydride adsorption, and combinations of two or more thereof.

The process for making at least one ethylene derivative in the first system illustrated by FIG. 1 may be performed as follows. The ethylene-containing fraction A (which exists a quench column - not shown) is first optionally passed through the upstream compressor 45 to increase the pressure of the resulting stream A1 to a pressure suitable for operating at least the oxychlorination reaction. The pressure after main compression may be at least 1.1 bars (110 kPa), at least 2 bars (200 kPa), at least 3 bars (300 kPa), or at least 4 bars (400 kPa). The pressure after compression may be at most 25 bars (2500 kPa), at most 20 bars (2000 kPa), at most 15 bars (1500 kPa), at most 10 bars (1000 kPa), or at most 7 bars (700 kPa), or at most 4 bars (400 kPa).

The fraction A or optionally-compressed stream A1 may be optionally subjected to desulfurization by passing through the upstream desulfurization unit 50 where sulfur compounds are at least partially removed.

The fraction A or optionally-compressed stream A1, either of which being optionally desulfurized, is split into two slipstreams B1 and B2 at split point 'T'. At least a portion of the slipstream B1, or preferably most or all of slipstream B1, is directed to the first (oxychlorination) reactor 80. At least a portion of the other slipstream B2, or preferably most or all of slipstream B2, is directed to the second (chlorination) reaction 90 after being treated in oxygenate removal unit 60 and drying unit 70. The removal of at least a portion of the oxygenates from the slipstream B2 in oxygenate removal unit 60 is carried out before drying in drying unit 70. Before the slipstream B2 or portion thereof is directed to units 60 and 70, it may be optionally sent to the optional downstream compressor 55 to increase the pressure of this stream to a pressure compatible with the operating pressure of the chlorination reaction in reactor 90. The pressure after such secondary compression may be at least 1.2 bars (120 kPa), at least 2 bars (200 kPa), or at least 4 bars (400 kPa). The pressure after secondary compression may be at most 25 bars (2500 kPa), or at most 20 bars (2000 kPa), or at most 15 bars (1500 kPa), or at most 10 bars (1000 kPa), or at most 6 bars (600 kPa).

The conditions in oxygenate removal unit 60 are such that an oxygenates-lean ethylene-containing stream 65 exits unit 60. At least a portion of the oxygenates-lean ethylene-containing stream 65 is directed to the drying unit 70. The conditions in drying unit 70 are such that an ethylene-containing feedstream C2 (which is leaner in water and oxygenates than slipstream B2) exits unit 70 and is directed to the second reactor 90. The reactor 90 preferably comprises a chlorination reaction in which at least some of the ethylene present in feedstream C2 is converted to DCE.

Optionally a portion 65' of the oxygenates-lean ethylene-containing stream 65 (exiting the oxygenates removal unit 60) is not directed to drying unit 70 but instead may be directed to the first reactor 80. The first reactor 80 which also receives the slipstream B 1 or portion thereof preferably comprises an oxychlorination reaction in which at least some of the ethylene originating from slipstream B 1 and optionally from stream 65' is converted to DCE.

Both product streams P1 and P2 exiting the first and second reactors 80 and 90, respectively, preferably contain DCE. Products streams P1 and P2 may additionally contain unconverted ethylene.

FIG. 2 illustrates a second system for carrying out the process according to the present invention, which is very similar to that of FIG. 1 in terms of optional upstream compression in unit 45, oxygenates removal in unit 60, and drying in unit 70, but instead of including an upstream desulfurization unit 50, this system may include an optional downstream desulfurization unit 95 for removing sulfur compounds from at least a portion of slipstream B1 and/or may include one or more optional downstream desulfurization units 120, 120" for removing sulfur from at least a portion of the slipstream B2.

The process for making at least one ethylene derivative carried out in the system illustrated by FIG. 2 comprises the following steps :
Step (f1): removing at least a portion of the oxygenates from slipstream B2 in oxygenates removal unit 60 before drying step (e) in drying unit 70; and
Step (i1): optionally carrying out a secondary compression on the slipstream B2 in compression unit 55 before the oxygenates removal step (f) in unit 60.

The process for making at least one ethylene derivative in the system embodied in FIG. 2 may be performed as follows. The ethylene-containing fraction A or optionally-compressed stream A1 is split into two slipstreams B 1 and B2, where at least a portion of the slipstream B2 is treated in oxygenates removal unit 60 and then in drying unit 70 before being directed to the second reactor 90, and where at least a portion of the other slipstream B 1 is directed to the first reactor 80.

One of the main differences between FIG. 2 compared to FIG. 1 is in the optional removal of sulfur compounds. The sulfur removal from one or more ethylene-containing streams in FIG. 2 may be carried out in one or more downstream desulfurization units after the split point T, whereas in FIG. 1, the sulfur removal from one or more ethylene-containing streams (fraction A or stream A1) may be carried out in an upstream desulfurization unit before the split point T. The process carried out in the system illustrated in FIG. 2 may include a desulfurization step selected from the group consisting of steps (j1), (j2), (j4), (j5), and combinations thereof.

The process for making at least one ethylene derivative in the system embodied in FIG. 2 may optionally comprise at least one of the following desulfurization steps.

For optional step (j1), the slipstream B2 or a portion thereof may be subjected to desulfurization (before drying in unit 70 and oxygenates removal in unit 60) in one or more optional downstream desulfurization units such as in downstream desulfurization unit 120 positioned downstream of the split point T and upstream of the oxygenates removal unit 60. The optional desulfurization step (j 1) in unit 120 comprises a desulfurization technique selected from the group consisting of an alkali wash, an activated carbon adsorption, an alkali metal hydride adsorption, and combinations of two or more thereof. Desulfurization in downstream desulfurization unit 120 may be done after the slipstream B2 is passed through the optional downstream compressor 55.

For optional step (j2), the slipstream B2 or a portion thereof may be subjected to desulfurization in a downstream desulfurization unit (not illustrated in FIG. 2) positioned between the oxygenates removal unit 60 and the drying unit 70; the desulfurization technique for optional step (j2) may be selected from the group consisting of an alkali wash, an activated carbon adsorption, an alkali metal hydride adsorption, and combinations of two or more thereof.

For optional step (j4), the feedstream C2 or a portion thereof may be subjected to desulfurization in one or more optional downstream desulfurization units such as in downstream desulfurization unit 120" (as illustrated) positioned downstream of the drying unit 70 and upstream of the second reactor 90. The optional step (j4) in desulfurization unit 120" comprises: subjecting the ethylene-containing feedstream C2 resulting from the drying step (e) in drying unit 70 and oxygenate removal step (f1) in unit 60 to a desulfurization technique which includes an activated carbon adsorption in desulfurization unit 120".

For optional step (j5), the slipstream B1 may be sent to an optional downstream desulfurization unit 95 before being directed to the first reactor 80. The optional step (j5) in desulfurization unit 95 comprises a desulfurization technique selected from the group consisting of an alkali wash, an activated carbon adsorption, an alkali metal hydride adsorption, and combinations of two or more thereof.

In alternate or additional variants, the system illustrated in FIG. 2 may further include the optional downstream compressor 55 so that at least a portion of slipstream B2 is compressed before entering the second reactor 90. The slipstream B2 may be compressed in the optional downstream compressor 55 before it is directed to units 60 and 70, and also before it is directed to optional desulfurization unit 120.

The optionally-desulfurized slipstream B 1 or a portion thereof is generally directed to the first reactor 80 which comprises an oxychlorination reaction in which at least some of the ethylene is converted to DCE.

At least a portion of the ethylene-containing stream 65 exiting the oxygenates removal unit 60 is directed to the drying unit 70.

At least a portion of the ethylene-containing feedstream C2 exiting the drying unit 70 may be directed to the desulfurization unit 120" before being sent to the second reactor 90. When the desuflurization step (j4) takes place in unit 120", at least a portion of the desulfurized ethylene-containing stream 125 exiting unit 120" is directed to the second reactor 90 for some of the ethylene contained herein to be converted to DCE.

Optionally a portion 65' of the ethylene-containing stream 65 exiting unit 60 and/or a portion 125' of the desulfurized ethylene-containing stream 125 exiting unit 120" may be directed to the first reactor 80 for some of the ethylene contained herein to be converted to DCE, at the same time as the ethylene originating from slipstream B 1 is converted to DCE in reactor 80.

Both product streams P1 and P2 exiting the first and second reactors 80 and 90 respectively contain DCE, and streams P1 and P2 may additionally contain unconverted ethylene.

FIG. 3 illustrates a third system for carrying out the process according to the present invention, which is similar to that of FIG. 2 in terms of optional upstream compression in unit 45, optional downstream compression in unit 55, and drying in unit 70, but instead of including separate optional downstream desulfurization units (in units 120, 120") and oxygenates removal in unit 60, this system includes a combined desulfurization and oxygenates removal unit 100 for removing sulfur and oxygenates simultaneously from the slipstream B2 or a portion thereof.The process for making at least one ethylene derivative carried out in the first system illustrated by FIG. 3 comprises the following steps:
Step (f1): removing at least a portion of the oxygenates from slipstream B2 in unit 100 before drying step (e) in unit 70; and

Optional Step (i1): optionally carrying out a secondary compression in optional downstream compression unit 55 on the slipstream (B2) before the oxygenate removal step (f) in unit 100 is carried out.

The process for making at least one ethylene derivative in the system embodied in FIG. 3 may be performed as follows. The ethylene-containing fraction A or compressed stream A1 is split into the two slipstreams B1 and B2, where at least a portion of the slipstream B2 is treated in a combined removal step for sulfur compounds and oxygenates in unit 100 and then is subjected to drying in drying unit 70 before being directed to the second reactor 90. At least a portion of the other slipstream B 1 is directed to the first reactor 80.

The process carried out in the system illustrated in FIG. 3 include the optional desulfurization step (j3) and may further include an optional desulfurization step (j5).

For optional step (j3), the slipstream B2 or a portion thereof is subjected to desulfurization (simultaneously to oxygenates removal) in unit 100 positioned downstream of the split point T and upstream of the drying unit 70. Desulfurization in unit 100 is preferably done after the optional secondary downstream compressor 55 and comprises a desulfurization technique selected from the group consisting of an alkali wash, an alkali metal hydride adsorption, and combination thereof.

For optional step (j5), similarly as described for FIG. 2, the slipstream B 1 may be sent to the optional downstream desulfurization unit 95 before being directed to the first reactor 80. The desulfurization technique in unit 95 is selected from the group consisting of an alkali wash, an activated carbon adsorption, an alkali metal hydride adsorption, and combinations of two or more thereof.

At least a portion of a purified stream 65 (which is preferably leaner in oxygenates and sulfur compounds than the slipstream B2 formed at point T) exiting the unit 100 is directed to the drying unit 70 to generate the feedstream C2 which is then sent to the second reactor 90 for some of the ethylene contained herein to be converted to DCE.

The slipstream B1, optionally desulfurized in unit 95, and optionally a portion 65'(which is not directed to reactor 90) of the purified ethylene-containing stream 65 exiting the unit 100 are directed to the first reactor 80 for some of the ethylene contained herein to be converted to DCE.

Both product streams P 1 and P2 exiting the first and second reactors 80 and 90 respectively contain DCE and may additionally contain unconverted ethylene.

FIG. 4 illustrates a fourth system for carrying out the present process according to the present invention, which includes the optional upstream compressor 45, the drying unit 70, a fractionation 110 which serves as an oxygenates removal unit, the first (oxychlorination) reactor 80, and the second (chlorination) reactor 90. In some variants, the system illustrated in FIG. 4 may further include an optional downstream desulfurization unit 95 for removing sulfur from slipstream B1, or one or more optional downstream desulfurization units (e.g., 120 or 120') for removing sulfur compounds from at least a portion of the slipstream B2; or an optional downstream desulfurization unit 120" for removing sulfur compounds from at least a portion of the feedstream C2.

The process for making at least one ethylene derivative carried out in the first system illustrated by FIG. 4 comprises step (f2) : removing at least a portion of oxygenates from a dried ethylene-containing stream after drying step (e) in unit 70. The removal of oxygenates preferably includes a fractionation in column 110. The slipstream B2 is sent to the drying unit 70 in which at least a portion of some water is removed to form the dried stream which is directed to the fractionation column 110. A light fraction exiting near or at the top of the fractionation column 110 contains most of the ethylene and provides the feedstream C2 which is directed at least in part to the second reactor 90. The fractionation column 110 further provides a heavy fraction 105 which contains the oxygenates which have been removed from slipstream B2.

In alternate or additional variants, the process for making at least one ethylene derivative carried out in the first system illustrated by FIG. 4 may further comprise the optional step (i2) : compressing the slipstream B2 in an optional secondary compression unit before the drying step and the oxygenates removal step carried out via fractionation.

The system illustrated in FIG. 4 may include an alternative or additional optional downstream compressor (not illustrated) downstream of the drying unit 70 but upstream of the second reactor 90. For example, the dried slipstream B2 may be compressed in such optional downstream compressor after it exits drying unit 70 but before it enters the fractionation column 110.

The process carried out in the system of FIG. 4 may further include an optional desulfurization step selected from steps (j1), (j2), (j4), (j5), and any combinations thereof.

For optional step (j1), the slipstream B2 or a portion thereof may be subjected to desulfurization (before drying in unit 70 and oxygenates removal in fractionation column 110) in a downstream desulfurization unit 120' positioned downstream of the split point T and upstream of the oxygenates removal unit 60.

For optional step (j2), the slipstream B2 or a portion thereof may be subjected to desulfurization in an optional downstream desulfurization unit 120" positioned in between the drying unit 70 and the fractionation column 110. downstream of the split point T and upstream of the oxygenates removal unit 60.

The optional desulfurization step (j1) in unit 120 or (j2) in unit 120' may comprise a desulfurization technique selected from the group consisting of an alkali wash, an activated carbon adsorption, an alkali metal hydride adsorption, and combinations of two or more thereof.

For optional step (j4), the ethylene-containing feedstream C2 or a portion thereof may be subjected to desulfurization in downstream desulfurization unit 120" positioned downstream of the fractionation unit 110 and upstream of the second reactor 90. The desulfurization technique in unit 120" may include an activated carbon adsorption. At least a portion of the desulfurized stream 125 which exits the downstream desulfurization unit 120" is directed to the second reactor 90, while another portion 125' may be directed to the first reactor 80.

The optional desulfurization step (j5) carried out in unit 95 is similar to the step described earlier in relation to FIG. 2 and 3.

FIG. 5 illustrates a fifth system for carrying out the process according to the present invention, which is similar to that of FIG. 4 in terms of optional upstream compression in unit 45, drying in unit 70, and fractionation in column 110 (as a means for oxygenates removal), but this system further includes a unit 60 for removing oxygenates from slipstream B2 or a portion thereof. This unit 60 is positioned downstream of the split point T and upstream of the drying unit 70. The system in FIG. 5 also differ from the system of FIG. 4 in that it includes an optional upstream desulfurization unit 50, rather than the optional downstream desulfurization units (120, 120', 120") shown in FIG. 4.

The system illustrated in FIG. 5 may include one or more optional downstream compressors (e.g., 55, 55'), so that at least a portion of slipstream B2 may be compressed before entering the second reactor 90. The one or more downstream compression unit 55, 55' is/are being positioned upstream of the fractionation column 110, and even preferably upstream of the drying unit 70. The slipstream B2 may be compressed in the optional downstream compressor 55 before it enters oxygenates removal unit 60 and/or may be compressed in the optional downstream compressor 55' after it exits oxygenates removal unit 60 but before it enters the fractionation column 110.

The process for making at least one ethylene derivative carried out in the first system illustrated by FIG. 5 comprises step (f3) : removing at least a portion of oxygenates from the slipstream B2 before drying step (e) and removing at least another portion of oxygenates from the dried ethylene-containing stream after drying step (e). The removal of oxygenates performed after drying preferably includes a fractionation in column 110.

The slipstream B2 which is optionally compressed in unit 55 is sent to the oxygenates removal unit 60 (where at least a portion of oxygenates is removed), is then sent after being optionally compressed in unit 55' to the drying unit 70 in which at least a portion of water is removed to form a dried stream which is then directed to the fractionation column 110 (where at least another portion of oxygenates is removed). The feedstream C2 is provided by a light fraction exiting near or at the top of the fractionation column 110 which contains most of the ethylene. The fractionation column 110 further provides a heavy fraction 105 which contains the oxygenates which are being removed. At least a portion 115 of feedstream C2 which exits the fractionation column 110 near or at the top is directed to the second reactor 90, while another portion 115' of feedstream C2 may be directed to the first reactor 80.

In alternate or additional variants, the process for making at least one ethylene derivative carried out in the system illustrated by FIG. 5 may further comprise optional step (i2) : performing a secondary compression in unit 55 and/or 55' on the slipstream B2 before the drying step (e) in unit 70 and before the oxygenates removal step (f) carried out via fractionation in column 110.

An optional desulfurization step (j') may be carried out in an upstream desulfurization unit before the split point T to remove some sulfur compounds from the fraction A or optionally-compressed stream A1 (as shown in FIG. 5) or to remove some sulfur compounds directly from fraction A before compression in upstream compressor 45 (not illustrated).

FIG. 6 illustrates a sixth system for carrying out the process according to the present invention, which is similar to that of FIG. 5 in terms of optional upstream compression in 45, drying in unit 60, and oxygenates removal in unit 60 and in fractionation column 110.

One of the main differences between FIG. 6 compared to FIG. 5 is in the optional removal of sulfur compounds. The sulfur removal from one or more ethylene-containing streams in FIG. 6 is carried out in a combined sulfur and oxygenates removal unit 100 or an optional desulfurization unit 95 after the split point T, whereas the sulfur removal from an ethylene-containing stream in FIG. 5 may be carried out in an optional upstream desulfurization unit 50 before the split point T. The process carried out in the system illustrated in FIG. 6 may include an optional desulfurization step (j3) and ,ay further include an optional step (j5).

For step (j3), the slipstream B2 or a portion thereof may be subjected to desulfurization (simultaneously to oxygenates removal) in unit 100 positioned downstream of the split point T and upstream of the drying unit 70. Desulfurization in unit 100 may be done downstream of the optional compression in secondary downstream compressor 55 and/or upstream of the optional compression in secondary downstream compressor 55'. Desulfurization in unit 100 comprises a desulfurization technique selected from the group consisting of an alkali wash, an alkali metal hydride adsorption, and combination thereof.

For optional step (j5), the slipstream B1 may be sent to the optional downstream desulfurization unit 95 before being directed to the first reactor 80, wherein the desulfurization technique in unit 95 is selected from the group consisting of an alkali wash, an activated carbon adsorption, an alkali metal hydride adsorption, and combinations of two or more thereof.

In alternate or additional variants, the process for making at least one ethylene derivative carried out in the system illustrated by FIG. 6 may further comprise optional step (i2): passing the slipstream B2 through secondary compression unit 55 or 55' before drying in unit 70 and before fractionation in column 110.

The process carried in FIG. 6 is similar to that described for FIG. 5 except that the slipstream B2 is sent to the combined sulfur and oxygenates removal unit 100 (where at least a portion of oxygenates and at least a portion of sulfur compounds are removed), before it is sent to the drying unit 70, and then directed to the fractionation column 110 (where at least another portion of oxygenates is removed).

FIG. 7 illustrates a seventh system for carrying out the process according to the present invention, which is similar to that of FIG. 5 in terms of optional upstream compression in unit 45, optional downstream compression in one or more units 55, 55', drying in unit 60, and oxygenates removal in two separate units: in unit 60 and in fractionation column 110.

One of the main differences between FIG. 7 compared to FIG. 6 is in the optional removal of sulfur compounds. The sulfur removal step in FIG. 7 may be carried out on feedstream C2 in desulfurization unit 120", whereas the sulfur removal step in FIG. 6 may be carried out on slipstream B2 in a combined sulfur and oxygenates removal unit 100.

The process carried out in the system illustrated in FIG. 7 may include a desulfurization step selected from steps (j4), (j5), and combinations thereof.

For optional step (j4), the ethylene-containing feedstream C2 or a portion thereof may be subjected to desulfurization in downstream desulfurization unit 120" positioned downstream of the fractionation unit 110 and upstream of the second reactor 90. The desulfurization technique in unit 120" may include an activated carbon adsorption. At least a portion of the desulfurized stream 125 which exits the downstream desulfurization unit 120" is directed to the second reactor 90, while another portion 125' may be directed to the first reactor 80.

The optional desulfurization step (j5) carried out in unit 95 is similar to the step described earlier in relation to FIG. 2 - 4, 6, 7.

FIG. 8 illustrates an eighth system for carrying out the process according to the present invention, which is similar to that of FIG. 7 in terms of optional upstream compression in unit 45, optional downstream compression in one or more units 55, 55', drying in unit 60, and two separate oxygenates removal units 60 and 110 (fractionation column). The process carried out in the system illustrated in FIG. 8 may include an optional desulfurization step selected from steps (j'), (j1) (j2), (j3) (j4), (j5), and combinations thereof.

One of the main differences between FIG. 8 compared to FIG. 1-7 is in the use of at least a portion P2' of the product stream P2 exiting the second reactor 90 (chlorination) as a feed to the first reactor 80. In this arrangement, the reactors 80 and 90 are operated in series, rather than operated in parallel as described in FIG. 1-7. This arrangement can be carried out when the product stream P2 still contains unconverted ethylene. The ethylene derivative (DCE) present in feedstream C2 may be removed in optional separation unit 130 before directing the DCE-lean portion P2' to the first (oxychlorination) reactor 80. The portion P2" which contains DCE (and which may be enriched in DCE after the optional separation in unit 130) may be sent to a third reactor (not shown), for the conversion of the ethylene derivative (DCE) to another compound, such as vinyl chloride (VC). Product stream P1 may be sent to the same third reactor to make VC or to another reactor to also make VC or to make another compound.

Although portion P2' of the product stream P2 is diverted to first reactor 80 in this particular embodiment, it is to be understood that in FIG. 1-7 a portion P2' of product stream P2 may be also diverted to reactor 80 in a similar manner as in FIG. 8 for the reactors 80 and 90 to be operated in series rather than parallel.

This disclosure of all patent applications, and publications cited herein are hereby incorporated by reference, to the extent that they provide exemplary, procedural or other details supplementary to those set forth herein.

While various embodiments may have been described independently from one another, it is to be understood that any combinations of two or more of such embodiments are further included in the present invention.

Should the disclosure of any of the patents, patent applications, and publications that are incorporated herein by reference conflict with the present specification to the extent that it might render a term unclear, the present specification shall take precedence.

In the application, where an element or component is said to be included in and/or selected from a list of recited elements or components, it should be understood that in related embodiments explicitly contemplated here, the element or component can also be any one of the individual recited elements or components, or can also be selected from a group consisting of any two or more of the explicitly listed elements or components. Further, it should be understood that elements and/or features of an apparatus, a process, or a method described herein can be combined in a variety of ways without departing from the scope and disclosures of the present teachings, whether explicit or implicit herein.

The use of the singular herein includes the plural (and vice versa) unless specifically stated otherwise. In addition, where the use of the term "about" is before a quantitative value, the present teachings also include the specific quantitative value itself, unless specifically stated otherwise. As used herein, the term "about" refers to a +-10% variation from the nominal value.

Each and every claim is incorporated into the specification as an embodiment of the present invention. Thus, the claims are a further description and are an addition to the preferred embodiments of the present invention.

While preferred embodiments of this invention have been shown and described, modifications thereof can be made by one skilled in the art without departing from the spirit or teaching of this invention. The embodiments described herein are exemplary only and are not limiting. Many variations and modifications of systems and methods are possible and are within the scope of the invention.

Accordingly, the scope of protection is not limited to the description of the embodiments described herein, but is only limited by the claims that follow, the scope of which shall include all equivalents of the subject matter of the claims.

## Claims

1. A process for the manufacture of at least one ethylene derivative compound from bioethanol, comprising:
a) subjecting a feedstock comprising biomass-derived ethanol to dehydration promoting conditions to produce an olefins stream comprising ethylene;
b) separating the olefins stream to form at least one fraction containing most of the ethylene (fraction A), said fraction A further comprising oxygenates and water;
c) optionally, compressing at least a portion of fraction A to form a compressed stream A1;
d) dividing said fraction A or said compressed stream A1 into a first slipstream B1 and a second slipstream B2;
e) passing at least a portion of said slipstream B2 through a drying unit to form a dried ethylene-containing stream;
f) performing an oxygenate removal step selected from the group consisting of:
f1) removing at least a portion of oxygenates from said slipstream B2 before
drying step (e);
f2) removing at least a portion of oxygenates from the dried ethylene-containing stream after drying step (e); and
f3) removing at least a portion of oxygenates from said slipstream B2 before
drying step (e) and removing another portion of oxygenates from the dried ethylene-containing stream after drying step (e),
wherein steps (e) and (f) are effective in generating an ethylene-containing feedstream C2, which is leaner in water and oxygenates than slipstream B2;
g) conveying the first slipstream B1 or a portion thereof to a first reactor to convert ethylene to at least one first ethylene derivative and to generate a first product stream P1 comprising said formed first ethylene derivative; and
h) conveying the ethylene-containing feedstream C2 or a portion thereof to a second reactor to convert ethylene to at least one second ethylene derivative and to generate a second product stream P2 comprising said formed second ethylene derivative,
wherein said second ethylene derivative formed in step (h) is optionally the same as the first ethylene derivative formed in step (g).

2. The process according to claim 1, wherein the first and second ethylene derivative compounds formed in steps (g) and (h) are the same and are 1,2-dichloroethane.

3. The process according to Claim 2, further comprising
(k) passing at least a portion of the 1,2-dichloroethane formed in step (g), at least a portion of the 1,2-dichloroethane formed in step (h), or any combination thereof through a third reactor under suitable 1,2-dichloroethane cracking conditions to generate vinyl chloride.

4. The process according to Claim 2, further comprises: mixing at least a portion of the 1,2-dichloroethane formed in step (g) and at least a portion of the 1,2-dichloroethane formed in step (h), and wherein the process further comprises:
(k) passing at least a portion of said mixture through a third reactor under suitable 1,2-dichloroethane cracking conditions to generate vinyl chloride.

5. The process according to Claim 3 or 4, further comprising:
(1) polymerizing the vinyl chloride produced in step (k) to produce polyvinyl chloride.

6. The process according to any of the preceding claims, comprising performing step (f1).

7. The process according to claim 6, further comprising:
(i1) carrying out a secondary compression on said slipstream (B2) before step (f1).

8. The process according to any one of Claims 1 to 5, comprising performing step (f2) or step (f3), and wherein the removal of oxygenates which is carried out in step (f2) or step (f3) after the drying step (e) includes a fractionation.

9. The process according to claim 8, further comprising:
(i2) carrying out a secondary compression step on said second slipstream B2 before fractionation in step (f2) or (f3) and before drying step (e).

10. The process according to claim 8, comprising performing step (f3), and wherein the removal of oxygenates after drying step (e) includes a fractionation, and wherein the removal of oxygenates before drying step (e) excludes a fractionation.

11. The process according to any of the preceding claims, wherein the product stream P2 comprises unconverted ethylene, and wherein at least a portion of the product stream P2 is directed to the first reactor in order for at least a part of the unconverted ethylene originating from said portion of product stream P2 to get converted to the first ethylene derivative.

12. The process according to any of the preceding claims, further comprising performing a desulfurization step (j') which comprises subjecting the fraction A or the compressed stream A1 to a desulfurization technique before forming the slipstreams B1 and B2, said desulfurization technique being selected from the group consisting of an alkali wash, a reaction with a peroxide, an activated carbon adsorption, an alkali metal hydride adsorption, and combinations of two or more thereof.

13. The process according to any of the preceding claims, further comprising performing a desulfurization step (j) after slipstreams B1 and B2 are formed, wherein said desulfurization step (j) comprises at least one of the following steps selected from the group consisting of:
(j1) subjecting the slipstream B2 or a portion thereof to a desulfurization technique selected from the group consisting of an alkali wash, an activated carbon adsorption, an alkali metal hydride adsorption, and combinations of two or more thereof, said step (j 1) being carried out before the drying step (e) and before the oxygenates removal step (f);
(j2) subjecting the slipstream B2 or a portion thereof to a desulfurization technique selected from the group consisting of an alkali wash, an activated carbon adsorption, an alkali metal hydride adsorption, and combinations of two or more thereof, said step (j2) being carried out in between the drying step (e) and the oxygenates removal step (f);
(j3) subjecting the slipstream B2 or a portion thereof during the oxygenates removal step (f1) to a desulfurization technique selected from the group consisting of an alkali wash, an alkali metal hydride adsorption, and combination thereof;
(j4) subjecting the ethylene-containing feedstream C2 resulting from step (e) and step (f) to a desulfurization technique which includes an activated carbon adsorption;
(j5) subjecting the slipstream B 1 to a desulfurization technique selected from the group consisting of an alkali wash, an activated carbon adsorption, an alkali metal hydride adsorption, and combinations of two or more thereof; and
any combinations of two or more thereof.

14. The process according to any of the preceding claims, comprising performing the compression step (c).

15. The process according to any of the preceding claims, wherein the compression step (c) is omitted.
